# EUROPEAN PATENT SPECIFICATION

(11) **EP 4 200 007 B1**
(45) Date of publication and mention of the grant of the patent: **18.03.2026**
(21) Application number: 21762733.0
(22) Date of filing: 23.08.2021
(51) Int. Cl.: A61N 1/04, A61N 1/372

(54) **TRAINING SYSTEM WITH INTERACTION ASSIST FEATURE, TRAINING ARRANGEMENT AND TRAINING**
TRAININGSSYSTEM MIT INTERAKTIONSUNTERSTÜTZUNGSFUNKTION, TRAININGSANORDNUNG UND TRAINING
SYSTÈME D'ENTRAÎNEMENT AVEC FONCTION D'AIDE À L'INTERACTION, AGENCEMENT D'ENTRAÎNEMENT ET ENTRAÎNEMENT

(30) Priority: 24.08.2020 EP 20461559
(43) Date of publication of application: 28.06.2023
(73) Proprietor: Neuro Device Group S.A., 01-510 Warsaw (PL)
(72) Inventor: GARNIER, Justyna, Julia, 01-510 Warszawa (PL); KINASIEWICZ, Joanna, 01-510 Warszawa (PL); MALEJ, Krzysztof Mateusz, 01-510 Warszawa (PL); SOLUCH, Pawel Sebastian, 01-510 Warszawa (PL)
(74) Representative: Schmidt, Christian
(86) International application number: PCT/EP2021/073222
(87) International publication number: WO 2022/043240

(56) References cited:
- US-A1- 2016 175 589

## Description

Document US 2016/175589 relates to a system and method for providing electrical stimulation to a brain region of a user comprising: providing a set of tasks to the user within a time window ; providing an electrical stimulation treatment to the brain region of the user in association with the time window; as the user performs each task of the set of tasks: at a biosignal detection module, receiving a signal stream characterizing a neurological state of the user; from the signal stream, identifying a neurological signature characterizing the neurological state associated with the task of the set of tasks; and modulating the electrical stimulation treatment to the user based upon the neurological signature, wherein an aggregated parameter of the electrical stimulation treatment provided to the user during the time window does not exceed a maximum limit. The method can additionally comprise: generating a comparison between a neurological metric derived from the neurological signature and a condition for each task in the set of tasks; providing the set of tasks to the user in a modified sequence, based upon at least one neurological signature identified; and providing a task-bounding stimulus to the user configured to facilitate consolidation of learned information and/or behavior.

A training system is specified. Moreover, a training arrangement and a training are specified One object to be achieved is to provide an improved training system. Further objects to be achieved are to provide an improved training arrangement and an improved training.

These objects are achieved, inter alia, by the subject-matter of claims 1, 12 and 14. Advantages embodiments and further developments are subject of the dependent claims and can be further extracted from the following description and the figures.

The training may be a language training. The training system may be a language training system. The training arrangement may be a language training arrangement. In other instance, the training may be a cognitive training. The training system may be a cognitive training system. The training arrangement may be a cognitive training arrangement.

The training may be used for treating speech and language disabilities, e.g. aphasia, as well as cognitive or social impairments connected to various disorders, e.g. neurological conditions like stroke, traumatic brain injury (TBI), neurodegenerative disorders, like Mild Cognitive Impairment (MCI), dementia (Alzheimer's Disease), Parkinson's Disease or neurodevelopmental disorders (e.g. Autism Spectrum Disorder). However, the training may explicitly be used as a non-therapeutic training, e.g. for improving language skills or cognitive skills.

Firstly, the training system is specified. The training system is configured to provide a training of a first user. The first user may be a patient with a language disability or a cognitive impairment. According to at least one embodiment, the training system comprises a computer program product comprising program code which, when loaded and executed on an electronic control unit, provides an operation control system. Additionally or alternatively, the training system may comprise an electronic control unit with a program code, wherein the program code, when executed on the electronic control unit, provides an operation control system. The program code may be a so-called App.

The electronic control unit preferably comprises a processor or processing engine. The computer program product may be a data carrier, such as a memory stick, a CD (Compact Disc), a magnetic storing hard disk (HD), an SSD (Solid State Device). The data carrier may be non-transitory data carrier. The computer program product may be a data stream (on wire or wirelessly transmitted, e.g. as digital and/or analog signals). The electronic control unit may be part of a notebook, a PC, a tablet, a mobile phone or a smart phone.

According to at least one embodiment, the operation control system is configured to control the training of the first user.

According to at least one embodiment, the operation control system comprises a plurality of training functionalities. The training functionalities may be functionalities of the computer program, e.g. classes of the computer program. The training functionalities may be individual submodules of the program code or the computer program. The training functionalities may communicate with each other, i.e. exchanging data or information. During the training or a training session, the training functionalities may be executed simultaneously and/or one after the other.

According to at least one embodiment, the training functionalities comprise a data generation functionality. The data generation functionality may be configured to produce user capability data. The user capability data may be indicative for the capability of the first user in content processing.

The term "content" includes but is not limited to: language related content, memory related content, executive related content, visuospatial related content, emotional content. For example, a content may be an expression, an information, a notion, a fact, a picture and so on. The term "content processing" particularly includes one or more or all of: content comprehension, content acknowledgement, response production related to the content. Particularly, content processing comprises language production and/or language comprehension. The response production includes, e.g., speaking and/or writing and/or indicating a preferred option among available options.

For example, the user capability data may be pretreatment patient condition, i.e. information on the type of disorder of the first user. The patient information may include, e.g., type and location of a brain damage, fMRI data, list of content for treatment. Additionally, the user capability data may comprise contents, e.g. expressions (like words or phrases) or notions or information, to be used/treated in the training of the first user. For example, the contents may comprise critical contents, e.g. critical expressions, which are contents known to be difficult for the first user to process. Additionally or alternatively, the contents may comprise noncritical contents, e.g. noncritical expressions, which are contents known to be processible by the first user. The noncritical contents may be contents which have already been trained by the first user. The user capability data may comprise cognitive load data determined during training of the first user. Cognitive load data may be data indicative of psychophysiological data or psychophysiological pattern of the first user correlated with his/her capabilities of performing tasks, as well as other activities related to the training.

Some user capability data may be produced before the first training session of the first user and/or after or during one or more training sessions. In other words, the data generation functionality may be configured to produce the user capability data at the beginning of the training, i.e. before the first training session has started, and/or may be configured to update the user capability data during the training. Any kind of data generated, produced or determined during the training, e.g. by using other training functionalities, may be used to generate user capability data with help of the data generation functionality. The data generation functionality may, in particular, be configured to cause the electronic control unit to store the user capability data in a memory.

According to at least one embodiment, the training functionalities comprise a presentation functionality. The presentation functionality may be configured to cause the electronic control unit to issue a presentation command depending on the user capability data in order to present a feature via a user interface. The feature may be related to the capability of the first user in content processing.

A presentation command issued depending on the user capability data means that the presentation command is issued or determined by considering or taking into account the user capability data. Thus, the presentation command and the associated presented feature, which may be called capability feature, may transport information of the user capability data. However, additionally, the presentation functionality may be configured to cause the electronic control unit to issue presentation commands independently of the user capability data and/or depending on other data or other information. In the following, further information/data, depending on which presentation commands, can be issued will be mentioned. The presentation functionality may be configured to cause the electronic control unit to issue a presentation command depending on any combination of these data and/or information.

The user interface via which the feature is presented may be operatively coupled to the training device. The interface may be configured to present the feature and/or to receive an input of a user and to convert this input into user data. Here and in the following, a user interface may comprise one or more or of all of: a display, e.g. a touch screen, a microphone, a loudspeaker, a tactile or haptic feedback element, a visible or audible alert element, a VR(Virtual Reality)-device, e.g. VR glasses, a AR(Augmented Reality)-device, e.g. AR glasses, a mouse, a keyboard. A user may input information via the user interface, e.g. by speaking into the microphone, by writing on the touch screen, by typing on a keyboard, by clicking a mouse etc.

Here and in the following, a feature presented by a user interface is particularly understood to be an information or element which can be perceived or noticed and/or understood by a user. E.g. the feature is a visual and/or acoustic feature. The feature may be displayed on a display of the user interface. An acoustic feature may be presented by a loudspeaker of the user interface. The feature may be presented to the first user or to another user.

The feature may be related to the capability of the first user in content processing. This means, in particular, that the feature is indicative for the capability of the first user in content processing. For example, the feature presents a task which the first user has to perform and adapted to the capability of the first user or the feature presents an information about the capability of the first user.

In at least one embodiment, the training system for a training of a first user comprises
a) a computer program product comprising program code which, when loaded and executed on an electronic control unit, provides an operational control system, and/or
b) an electronic control unit with a program code, wherein the program code, when executed on the electronic control unit, provides an operation control system. The operation control system is configured to control the training of the first user and comprises a plurality of training functionalities. The training functionalities comprise a data generation functionality configured to produce user capability data, wherein the user capability data are indicative for the capability of the first user in content processing. Furthermore, the training functionalities comprise a presentation functionality which is configured to cause the electronic control unit to issue a presentation command depending on the user capability data in order to present a feature via a user interface. The feature may be related to the capability of the first user in content processing.

The training of the first user may comprise or consist of a plurality of training sessions. Each training session may have a duration of at least 1 minute or at least 10 minutes or at least 30 minutes. Additionally or alternatively, each training session may have a duration of at most 5 hours or at most 3 hours or at most 1 hour. Several training sessions may be performed on the same day. Different training sessions may be performed on different days. Between different training sessions, the training may be interrupted, for example for at least 10 minutes or at least 1 hour. During one or more or all training sessions, at least one, e.g. at least two or at least five or at least ten, presentation commands may be issued by the presentation functionality and according numbers of features may be presented via a user interface.

According to at least one embodiment, the training functionalities comprise a recognition functionality which is configured to recognize a content, e.g. an expression, particularly a critical content, e.g. a critical expression, depending on user input data provided with help of a user interface. The user data may be audio user input data. The interface may be an audio user interface. The input data are indicative for an input, e.g. a speech input, of a user, e.g. of the first user or another user. A critical content is a content the first user has problems to process.

The content may be an expression, e.g. a single word or a whole phrase or sentence. The user interface may comprise a microphone. A user speaks into the user interface, e.g. a microphone, (speech input) and this is transferred into audio user input data, from which the recognition functionality extracts one or more expressions. The recognition may preferably be performed in real-time. The recognition functionality may use a speech recognition software, e.g. a commercial speech recognition software.

According to at least one embodiment, the presentation functionality is configured to cause the electronic control unit to issue a presentation command depending on the recognized content, e.g. critical content, in order to present an assist feature via a user interface. The assist feature is configured to assist the first user in the training, e.g. assisting the first user to process the critical content.

The assist feature is a special type of feature presented via the user interface. It may be an audible and/or visual feature. For example, the assist feature is a synonym and/or a reformulation, e.g. presented in written or acoustic form, and/or a graphical depiction, e.g. a pictogram or a picture, of the (critical) content. The assist feature may also be a context related content, e.g. presented in written or acoustic of graphical form. The assist feature may also be a feature indicating to a second user that the first user has problems to process the recognized (critical) content, e.g. by highlighting the content.

According to at least one embodiment, the recognition functionality is configured to extract information out of the user input data, wherein the information is indicative for the topic. i.e. the context, of the user input. In other words, the recognition functionality may be configured to perform a semantic analysis depending on the user input data in order to extract the topic of the user input.

According to at least one embodiment, the presentation functionality is configured to cause the electronic control unit to issue a presentation command depending on the topic of the user input in order to present an assist feature via a user interface. For example, the assist feature is a critical or noncritical content, like an expression, presented in visible or audible form, which is related to the topic.

According to at least one embodiment, the recognition functionality is configured to recognize the content, particular the critical content, depending on user input data provided with help of user interface and being indicative for an input of a second user. The second user is different from the first user. For example, the second user may be an interlocutor, such as a family member or friend or therapist of the first user.

The first user may be assigned a first user interface and the second user may be assigned a second user interface. The first and the second user interface may be operatively coupled to the training system or may each be coupled to an individual training system, wherein the individual training systems may then be coupled to each other. The user interfaces may each be configured to present a presentation feature depending on a presentation command and/or to receive a user input by the assigned user and to convert it into user input data.

The first user interface may be unambiguously assigned to the first user and/or the second user interface may be unambiguously assigned to the second user. For example, the first user cannot perceive what is presented via the second user interface and/or the second user cannot perceive what is presented via the first user interface. The first and the second user interface may be located in different rooms and/or may be spaced from one another by more than 10 m or more than 100 meter or more than 1 km.

According to at least one embodiment, the user capability data are indicative for one or more critical contents known to make problems to the first user to process.

According to at least one embodiment, the recognition functionality is configured to recognize the critical content, e.g. the critical expression, depending on the user input data, e.g. the audio user input data, and the user capability data. The recognition functionality may extract one content after the other out of the user input data and may compare each extracted content with the critical content(s) of the stored user capability data. If an extracted content matches with a critical content of the user capability data, the extracted content is recognized / tagged as this critical content.

According to at least one embodiment, the recognition functionality is configured to recognize at least one critical content, e.g. critical expression, only depending on the user input data. For example, the recognition functionality recognizes that the first user has problems to process a content, e.g. is stuttering an expression. The recognition functionality may be configured such that it performs a semantic analysis of the user input and determines the content which the first user has problems to process depending on this semantic analysis. The determined content is then recognized/tagged as a critical content.

According to at least one embodiment, the presentation functionality is configured to cause the electronic control unit to issue a first presentation command, e.g. depending on the user capability data, in order to present a list of critical contents, e.g. critical expressions, via a user interface to indicate contents the first user has problems to process. The critical contents might be contents which should be trained by the first user in the training in order to improve the capabilities of the first user. Alternatively, the critical contents may be contents which should be avoided in the training. For example, the list of critical contents can be presented on the user interface in a table and for each critical content a synonym or a rephrasing or a graphical depiction may be presented in this table.

According to at least one embodiment, the presentation functionality is configured to cause the electronic control unit to issue a second presentation command, e.g. depending on the user capability data, in order to present a list of noncritical contents, e.g. noncritical expressions, via a user interface to indicate contents the first user is able to process. The noncritical contents may be contents which should be further used in the training, e.g. in order to improve the capability of the first user or in order to strengthen the self-confidence of the first user.

According to at least one embodiment, the first presentation command and/or the second presentation command are configured to present the list of critical and/or noncritical contents via a user interface for a second user (second user interface).

According to at least one embodiment, the presentation functionality is configured to be executed before the recognition functionality is executed. For example, the first and/or second presentation command are issued before the recognition functionality is executed.

Additionally or alternatively, the presentation functionality may be executed after the recognition functionality. It is also possible to execute the recognition functionality and the presentation functionality in an alternating manner. For example, every time a critical content is recognized, the presentation functionality issues a presentation command depending on the recognized critical content in order to present an assist feature via a user interface.

In one embodiment, every time the recognition functionality performs a semantic analysis of the user input data, e.g. an expression, the presentation functionality causes the electronic control unit to issue a presentation command in order to present the list of critical and/or noncritical contents via a user interface for a second user (second user interface), wherein the critical and/or noncritical contents are semantically related to the user input data.

According to at least one embodiment, the presentation functionality is configured to cause the electronic control unit to issue a presentation command, e.g. depending on the user capability data, in order to present a cognitive task via a user interface, particularly via the first user interface. The cognitive task is a task to be performed by the first user. A cognitive task may be any one of: visuospatial processing task, concentration task, attention task, planning task, problem solving task, memory task, repetition tasks, task in which a question must be answered, task which requires the description of the content of a picture, task in which a game, e.g. a computer game in the n-back or digit span paradigm must be played. The task may be chosen depending on the user capability data.

According to at least one embodiment, the training functionalities comprise a performance assessment functionality. The performance assessment functionality may be configured to determine, particularly to calculate or to provide, task performance data which is indicative for the performance of the first user in solving a task. For example, for performing the task, the first user has to perform an input via the first user interface, e.g. he/she has to write on the first user interface or has to speak into the first user interface or a has to press buttons of the first user interface.

The task may be a cognitive task and/or training task (see explanation further below). The task performance data may comprise a score value indicative for the performance of the first user in solving the task. For example, the score value can be an integer between 0 and 100, wherein a score value of 100 indicates a high task performance and a score value of 0 indicates a low task performance. Also, the time, e.g. reaction time, for the response to the task may be taken into account.

According to at least one embodiment, the training functionalities comprise a cognitive load functionality configured to determine cognitive load data depending on the task performance data, wherein the cognitive load data are indicative for the cognitive load (also called cognitive state in the following) of the first user. Additionally or alternatively, the cognitive load functionality may be configured to determine cognitive load data depending on psychophysiological data. Psychophysiological data are indicative for at least one psychophysiological measurement performed on the first user using a detector, e.g. a condition monitoring device. By way of example, the cognitive load of the first user can be classified into the following categories: distraction, low load, moderate load, high load and overload. Based on the cognitive load data, a cognitive load/state may be assigned to the first user. The cognitive load functionality is preferably individualized for each user.

According to at least one embodiment, the operation control system is configured to control the training depending on the determined cognitive load of the first user or depending on the determined cognitive load data, respectively. For example, the difficulty or level of following tasks (training tasks or cognitive tasks) generated with help of the presentation functionality is determined or changed depending on the cognitive load of the first user. Additionally or alternatively, a presentation command may be issued in order to present the second user the information of the cognitive load of the first user via the second user interface. A break may be suggested to the first user by the operation control system, if, e.g., high cognitive load is detected.

Preferably, the operation control system is configured to control the training depending on the determined cognitive load or load data, respectively, before the actual cognitive load of the first user changes, e.g. before overload occurs. For this purpose, the cognitive load data may be indicative for an upcoming change in the cognitive load.

Psychophysiological data may comprise one or more or all of the following indicators: emotional data, facial expressions data, electrodermal activity data, heart rate (variability) data, pulse data, microsaccades data, saccades data, pupillometry data, fixations and saccade data, blink data, voice recording data, declarative emotional state data, declarative work load data, declarative level of energy, breath duration data, breath frequency data, mouse trajectory data, mouse click data, body temperature data. The cognitive load data may be determined depending on all these indicators, only some of the indicators or only one of the indicators. Alternatively, in order to individualize performance, the operation control system may classify one or more of the above-mentioned indicators as irrelevant based on history of user data (e.g. if one of the indicators in the past sessions suggested breaks which were always skipped without negative influence on efficacy of the training and cognitive load was always quickly decreasing, the given indicator importance may be decreased). More than one indicator may be used to more precisely detect change of load, e.g. interaction of two indicators to detect cognitive load that is dominating over physiological arousal, e.g. analysis of pupil dilation signal in moments of heart rate increase.

One or more of the detectors / condition monitoring devices mentioned in the following may be used to generate the indicators: Emotional data and facial expressions data may be generated using a web-cam filming the first user. Electrodermal activity data and/or heart rate (variability) data and/or pulse data may be generated using a bracelet or watch worn by the first user. Microsaccades data and/or pupillometry data and/or fixations and saccade data may be generated using a video-based eye tracker and/or a web-cam eye tracker. Voice recording data, breath duration data, breath frequency data may be generated with help of a microphone. Declarative emotional state data and/or declarative workload data and/or declarative level of energy may be generated by the user answering a questionnaire, e.g. using NASA-TLX, and/or by a semantic analysis. This might require input, of the first user via the first user interface, e.g. speech or writing or pressing buttons. Body temperature data may be generated using a thermometer.

Eye tracking may be based on, e.g., Task-Evoked Pupillary Response (TEPR), inter-trial change in pupil diameter (BCPD), intra-trial change in pupil diameter (CPD), microsaccade rate, microsaccade magnitude, indicators of focal/ambient visual attention.

At least some of the detectors or condition monitoring devices mentioned above might be part of the first user interface. Particularly, this might be the case for a microphone and/or webcam.

For determining cognitive load data depending on the psychophysiological data, the cognitive load functionality may use a classifier. The classifier receives as an input task performance data and/or psychophysiological data and generates as an output cognitive load data or the cognitive load of the first user. The classifier may be an AI (Artificial Intelligence) based classifier. For example, the classifier comprises a neural network.

The classifier may be trained as follows: The first user may be presented several tasks, e.g. cognitive tasks. The corresponding task performance data may be considered as being primary indicative for the cognitive load of the first user. The extracted task performance data are then used to calibrate the psychophysiological indicators of cognitive load during cognitive processing. Tasks, particularly cognitive tasks, may be presented to the user at different levels of difficulty so the training system can recognize patterns of psychophysiological indicators and match them into the different classes of cognitive states/loads, e.g. distraction, low load, moderate load, high load and overload. Classification may be done on the basis of accuracy of responses and reaction times analysis when performing tasks. Furthermore, the training system may also collect data about psychophysiological activity, i.e. psychophysiological data, while the first user conducts training tasks (see explanation below). In this way, the classifier learns which psychophysiological data/pattern is indicative for which cognitive state of the first user.

Later on, the cognitive load functionality may extract psychophysiological patterns from the psychophysiological data and may use the psychophysiological pattern to determine the cognitive load data using the classifier. For example, the cognitive load functionality may then determine the cognitive load data or the cognitive load, respectively, depending only on psychophysiological data, i.e. without using task performance data.

The classifier may be trained during the training of the first user or after a training session. According to at least one embodiment, the cognitive load functionality is configured to determine cognitive load data depending on self-report by the first user. This might be a feature of the cognitive load functionality additionally or alternatively to determining cognitive load data depending on task performance data and/or psychophysiological data. For example, the operation control system causes the electronic control unit to issue a command, e.g. with help of the presentation functionality, in order to ask the first user to indicate his/her cognitive state. A corresponding feature/question may be presented via the user interface. Depending on the associated answer of the first user (self-report), the cognitive load functionality determines the cognitive load. The self-report of the first user can be answering a NASA-TLX questionnaire. The self-report of the first user may be speech input of the first user before, during or after solving a task, indicating how difficult the first user finds the task. Particularly, the self-report is a user input provide by the first user via a user interface, which generates self-report data and depending on the self-report data, the cognitive state may be determined.

A method of how to assess cognitive load from psychophysiological measures is described in the paper by Eija Haapalainen et al., "Psycho-Physiological Measures for Assessing Cognitive Load", Behavioural Brain Research 293 (2015) 2 UbiComp '10: Proceedings of the 12th ACM international conference on Ubiquitous computing, September 2010, Pages 301-310.

In the paper by Henrik Wiberg et al., "Physiological responses related to moderate mental load during car driving in field conditions", Biological Psychology, Volume 108, May 2015, Pages 115-125, multiple indicators, like heart-rate, skin conductance level, breath duration, blink frequency, blink duration, and eye fixation related potential and self-reporting were used to determine the cognitive load.

In the paper by Charlotte Larmuseau et al., "Combining physiological data and subjective measurements to investigate cognitive load during complex learning", Frontline Learning Research, 7(2), May 2019, Pages 57-74, it was studied how physiological data, namely electrodermal activity (EDA) is related to mental effort.

In the paper by Ramtin Zargari Marandi et al., "Reliability of Oculometrics During a Mentally Demanding Task in Young and Old Adults", IEEE Access, vol. 6, 2018, Pages 17500-17517, the sensitivity of oculometrics to changes in mental load is investigated.

In the paper by Krzysztof Krejtz et al., "Eye tracking cognitive load using pupil diameter and microsaccades with fixed gaze" PLOS ONE 13(9): e0203629, 2018, cognitive load is estimated based on measurement of microsaccades during mental calculation tasks.

In the paper by Petar Jerčié et al., "Modeling cognitive load and physiological arousal through pupil diameter and heart rate", Multimed Tools Appl 79, 2020, Pages 3145-3159, individuals' cognitive load processing abilities while engaged on a decision-making task in serious games is investigated, to explore how a substantial cognitive load dominates over the physiological arousal effect on pupil diameter.

User's task performance may be also influenced by its emotional state. The cognitive load functionality may also use indicators used for assessment of emotional processing in addition to determining the cognitive load (data).

According to at least one embodiment, the training functionalities comprise a stimulation functionality which is configured to cause the electronic control unit to issue a stimulation command to an electrical brain stimulation device to cause the electrical brain stimulation device to perform an electrical brain stimulation procedure.

According to at least one embodiment, the presentation functionality is configured to cause the electronic control unit to issue a presentation command, e.g. depending on the user capability data, in order to present a training task to be performed by the first user via a user interface.

The training task may be a language task or a cognitive task. The training task may comprise the presentation of words or sentences, pictures, pictograms, etc., preferably supported by non-invasive brain stimulation synchronized with specific task groups. To solve the task the first user may need to comprehend presented words or sentences, to recognize the content of the picture or pictogram and/or to actively produce language related output in speech or writing, particularly via the first user interface. Different kinds of tasks comprise repetitions, answering of questions, descriptions of the content of pictures or supply of semantically related words, e.g. use-generation task. A created training task extension may involve out-loud controlled dialogue with a chatbot, wherein the first user may pick one of several possibilities of response.

Preferably, the stimulation functionality and the presentation functionality may be linked or may be linkable to one another. The electrical brain stimulation procedure may be a transcranial stimulation procedure.

The stimulation functionality and the presentation functionality may especially be timely linked or synchronized, e.g. such that the brain stimulation procedure is performed while tasks (cognitive tasks and/or training tasks) are presented to and/or performed by the first user. The brain stimulation procedure may be performed for all tasks presented during a training session or only for selected or special tasks, e.g. tasks that are more difficult than other tasks presented in the same training session. By means of electrical brain stimulation the training system may positively influence the activity of neurons in the stimulated part of the brain. Expediently, the stimulated part(s) or region(s) of the brain is active when performing the task and/or used to solve the tasks that are presented by the presentation functionality. During a training combined with stimulation session, neuronal network strengthening may efficiently support a therapy processes and the first user may improve the brain function, e.g. language processing and/or speech generation ability, cognitive functions, memory performance etc., in a comparably short time due to this double influence to the neurons, e.g. by electrical stimulation and the natural brain activations involved in performing the task. The user's performance may be improved due to the additional electrical brain stimulation while presenting and/or performing the task. The linking of brain stimulation to the tasks which are performed may have a positive influence on, for example: the training effect for the brain, i.e. neuroplasticity, and/or the user's motivation to do several training sessions. The proposed system may be particularly suitable for users suffering from aphasia, e.g. after having suffered a stroke or a traumatic brain injury (TBI).

Additionally or alternatively, the stimulation functionality and the presentation functionality may especially be linkable or linked, e.g. time-synchronized, such that for instance a preparation stimulation is performed before the first task of the current training session is performed and/or presented. The preparation stimulation may be provided as an alternative or in addition to a task stimulation, which is performed during presentation and/or performance of the task. The plasticity of relevant brain areas may be increased by the preparation stimulation. Thus, the training efficiency may be improved even more.

In other words, the term "linked" may mean that the scheduling of the stimulation functionality and of the presentation functionality is coordinated with regard to each other, for instance by the operation control system. Scheduling refers for instance to at least one of: the time of the beginning of the stimulation procedure, the time of the beginning of the presentation of tasks, the time of terminating the stimulation procedure, and the time of terminating the presentation of tasks.

The term "linked" may further mean that the kind of stimulation and/or the intensity of stimulation, e.g. amplitude, duration, etc., may depend on the kind of task that has to be performed.

Thus, there is preferably an electrical brain stimulation functionality of specific brain areas used in combination, for instance also at the same time, with a presentation functionality that excites or activates the same brain areas of the user through physiological brain activity processes, in particular without external electrical stimulation. The combination of these two functionalities allows synergistic effects between both functionalities that are not possible if only one of the procedures is used or if there is no linkage between both functionalities.

The electrical brain stimulation procedure may be performed transcranially on the user, e.g. by using at least one electrode, two electrodes or a plurality of electrodes that are connected and/or fixed to the head of the first user of the brain stimulation system. The first user may be a patient that suffers from aphasia or other neurological disorders, such as cognitive impairment. Alternatively, the first user may be a healthy person that intends to improve his or her language processing and/or production skills or cognitive functions.

The type of stimulation in the stimulation procedure, e.g. in the preparation stimulation and/or the task stimulation, may be specified in the stimulation command to be transcranial electrical stimulation in constant current mode (transcranial direct current stimulation (tDCS)) or in varying current mode (e.g. transcranial alternating current stimulation (tACS), transcranial random noise stimulation (tRNS), pulses of current) or a sequence thereof.

The preparation stimulation may be a constant current stimulation. The amplitude of constant or direct current (DC) may be in the range of 0.1 mA to 10 mA or 0.1 to 5 mA, preferably 2 mA. Alternatively or additionally the amplitude of the direct current may be greater than or equal to one of the following values: 0.1, 0.2, 0.3, 0.4, 0.5, 0.6, 0.7, 0.8, 0.9, 1, 2, 3, 4, 5 mA. Alternatively or additionally, the amplitude of the direct current may be less than or equal to one of the following values: 10, 9, 8, 7, 6, 5 mA. All combinations between lower limits and upper limits are possible, for instance 1 to 7 mA. Thus, ranges may be formed by combining values from the lower limits and from the upper limits.

Alternatively, the preparation stimulation may be a varying current stimulation. For example, tRNS may be particularly suitable for a preparation stimulation in the varying current mode.

The task stimulation may preferably be a varying current stimulation. "Varying" may mean that the current is non-constant, preferably sinusoidal. The frequency of the stimulation, e.g. the frequency of stimulation in the specific brain area and/or the frequency of the varying current, may be in the range of 13 Hz (hertz) to 24 Hz, especially 17 Hz. Beta oscillation, e.g. in the range of 12.5 Hz to 30 Hz, may be preferred for the treatment of aphasia. Alternatively or additionally, the frequency may be greater than or equal to one of the following values: 12, 13, 14, 15, 16, 17, 18, 19 Hz. Alternatively or additionally, the frequency may be less than or equal to one of the following values: 25, 24, 23, 22, 21, 20, 19 Hz (Hertz). All combinations between lower limits and upper limits are possible, for instance 12 to 22 Hz. Thus, ranges may be formed by combining values from the lower limits and from the upper limits.

It has been shown, that beta frequency tACS (20 Hz) are most efficient in boosting neuroplastic effects in motor cortex (motor learning) - see Pollok et al., "The effect of transcranial alternating current stimulation (tACS) at alpha and beta frequency on motor learning", Behavioural Brain Research 293 (2015) 234-240. In Wernicke's area, similarly to motor cortex, there are also present oscillations of beta frequency. Specifically, it has been shown, that 17 Hz frequency is one of the most pronounced in Wernicke's activity (see Nikolaev et al., "Correlation of brain rhythms between frontal and left temporal (Wernicke's) cortical areas during verbal thinking", Neuroscience Letters 298 (2001) 107-110). Without to be bound by theory, based on that we hypothesized that 17 Hz stimulation may be particularly efficient for aphasia therapy.

The task stimulation may be performed with a frequency in range of 40 Hz and 50 Hz, e.g. at 40 Hz or at 50 Hz. It has been shown that using this frequency range or these frequencies has an influence on treatment of Alzheimer's disease (e.g. Dhaynaut, M., Pascual-Leone A., Santarnecchi E., El Fakhri G. (2020) "Effects of modulating gamma oscillations via 40Hz transcranial alternating current stimulation (tACS) on Tau PET imaging in mild to moderate Alzheimer's Disease", Journal of Nuclear Medicine 61(340). Without to be bound by theory, based on that we hypothesized that this frequency range may be efficient also in MCI and it would be beneficial to synchronize this type of stimulation with cognitive tasks.

However, additionally or alternatively, other frequency ranges may also be relevant, for instance alpha oscillation, as indicated in scientific papers, e.g. in the frequency range of 7.5 Hz to 12.5 Hz. The frequency may be greater than or equal to one of the following values: 7, 8, 9 Hz. The frequency may be less than or equal to one of the following values: 13, 12, 11, 10, 9 Hz. All combinations between lower limits and upper limits are possible, for instance 8 to 11 Hz.

Other frequencies may be also relevant, for instance delta (1-4 Hz), theta (4-8 Hz), low gamma (30-70 Hz) and high gamma (70-150 Hz). In particular, a frequency of 75 Hz may be used or a frequency within the range of 70 Hz to 80 Hz or of 60 Hz to 100 Hz or of 60 Hz to 250 Hz.

The following alternatives may be used for the frequency of the stimulation, e.g. preparation stimulation and/or task stimulation:
- alternative b), the frequency of varying current may be or is in the range of 70 Hz (hertz) to 80 Hz, e.g. 75 Hz,
- in the alternative b), the frequency of varying current is preferably greater than or equal to one of the following values: 69, 70, 71, 72, 73, 74, 75, 76, 77, 78, 79 Hz,
- in the alternative b), the frequency of varying current is preferably less than or equal to one of the following values: 80, 79, 78, 77, 76, 75, 74, 73, 72 or 71 Hz,
- alternative c), the frequency of varying current may be or is in the range of 60 Hz (hertz) to 100 Hz, e.g. 75 Hz or 80 Hz,
- in the alternative c), the frequency of varying current is preferably greater than or equal to one of the following values: 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 88 or 99 Hz,
- in the alternative c), the frequency of varying current is preferably less than or equal to one of the following values: 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62 or 61 Hz,
- alternative d), the frequency of varying current may be or is in the range of 60 Hz (hertz) to 250 Hz, e.g. 60, 70, 80, 90, 100, 110, 120, 130, 140, 150, 160, 170, 180, 190, 200, 210, 220, 230, 240 or 250 Hz,
- in the alternative d), the frequency of varying current is preferably greater than or equal to one of the following values: 60, 70, 80, 90, 100, 110, 120, 130, 140, 150, 160, 170, 180, 190, 200, 210, 220, 230, 240 Hz,
- in the alternative d), the frequency of varying current is preferably less than or equal to one of the following values: 250, 240, 230, 220, 210, 200, 190, 180, 170, 160, 150, 140, 130, 120, 110, 100, 90, 80 or 70 Hz,
- alternative e), the frequency of varying current may be or is in the range of 30 Hz (hertz) to 70 Hz, e.g. 30, 35, 40, 45, 50, 55, 60, 65 or 70 Hz,
- in the alternative e), the frequency of varying current is preferably greater than or equal to one of the following values: 30, 35, 40, 45, 50, 55, 60 or 65 Hz,
- in the alternative e), the frequency of varying current is preferably less than or equal to one of the following values: 70, 65, 60, 55, 50, 45, 40 or 35 Hz,
- alternative f), the frequency of varying current may be or is in the range of 70 Hz (hertz) to 150 Hz, e.g. 70, 75, 80, 85, 90, 95, 100, 105, 110, 115, 120, 125, 130, 135, 140, 145 or 150 Hz,
- in the alternative f), the frequency of varying current is preferably greater than or equal to one of the following values: 70, 75, 80, 85, 90, 100, 105, 110, 115, 120, 125, 130, 135, 140 or 145 Hz,
- in the alternative f), the frequency of varying current is preferably less than or equal to one of the following values: 150, 145, 140, 135, 130, 125, 120, 115, 110, 105, 100, 95, 90, 85 , 80 or 75 Hz.

The amplitude of the varying current stimulation may be in the range of 0.1 mA to 10 mA or 0.1 to 5 mA, preferably 2 mA. Alternatively or additionally, the amplitude may be greater than or equal to one of the following values: 0.1, 0.2, 0.3, 0.4, 0.5, 0.6, 0.7, 0.8, 0.9, 1, 2, 3, 4, 5 mA (milliampere). Alternatively or additionally, the amplitude may be less than or equal to one of the following values: 10, 9, 8, 7, 6, 5 mA. All combinations between lower limits and upper limits are possible, for instance 1 to 7 mA. Thus, ranges may be formed by combining values from the lower limits and from the upper limits.

According to at least one embodiment, the stimulation functionality comprises a task stimulation functionality for a task stimulation. The task stimulation may be configured to be synchronized with the presentation of the task (training task and/or cognitive task). The task stimulation may be performed during the presentation of the task to the first user and/or during the performance of the task by the first user.

According to at least one embodiment, the training functionalities comprise an environment functionality which is configured to receive environment user input data. The environment user input data is indicative for a user selected virtual environment at which the first user performs the training. For example, the user can select the virtual environment by pressing one of a plurality of buttons, e.g. on the user interface, wherein each button is assigned a different virtual environment.

According to at least one embodiment, the presentation functionality is configured to cause the electronic control unit to issue a presentation command in order to present an environment feature on a user interface. The environment feature is indicative for the user selected virtual environment.

The environment functionality may be executed before the recognition functionality. The environment feature may be a picture of an environment, e.g. forming a display background. Additionally or alternatively, the environment feature may be a at least one picture of subjects typical for the environment and/or at least one typical expression for the environment. The environment feature may be an animated room or a virtual reality space.

According to at least one embodiment, the presentation functionality is configured to cause the electronic control unit to issue a presentation command depending on the environment user input data. For example, the first and or second presentation command for presenting the list of contents is selected depending on the environment user input data. The critical and noncritical contents may be contents related to the selected virtual environment.

According to at least one embodiment, the data generation functionality is configured to produce, i.e. generate or update, the user capability data depending on an input provided with help of a user interface and performed by the user. For example, the first user and/or the second user may select contents, for instance expressions, on the assigned user interface, the contents being contents which, during a training session, the first user had problems to process or which the first user was able to process. This selection may confirm or may complement the list of critical contents and/or noncritical contents.

According to at least one embodiment, the generation functionality is configured to produce the user capability data depending on the task performance data. The task performance data may be indicative for which content has to be further trained or which content the first user can easily process.

In at least one embodiment, the training system for a training of a first user comprises
a) a computer program product comprising program code which, when loaded and executed on an electronic control unit, provides an operation control system, and/or
b) an electronic control unit with a program code, wherein the program code, when executed on the electronic control unit, provides an operation control system,
   - wherein the operation control system is configured to control the training of the first user,
   - wherein the training comprises a step in which a social interaction module is executed in which the first user interacts, particularly communicates, with a second user, e.g. via at least one user interface
   - wherein the operation control system comprises a plurality of training functionalities, the training functionalities comprising:
   - a recognition functionality which is configured to recognize a critical content depending on user input data provided with help of a user interface and being indicative for an input of either the first user or the second user, wherein the critical content is a content the first user has problems to process,
   - a presentation functionality which is configured to cause the electronic control unit to issue a presentation command depending on the recognized critical content in order to present an assist feature via a user interface, the assist feature being configured to assist the first user in processing the critical content.

In at least one embodiment, the training system for a training of a first user comprises:
a) a computer program product comprising program code which, when loaded and executed on an electronic control unit, provides an operation control system, and/or
b) an electronic control unit with a program code, wherein the program code, when executed on the electronic control unit, provides an operation control system,
   - wherein the operation control system is configured to control the training of the first user,
   - wherein the training comprises a step in which a social interaction module is executed in which the first user interacts with a second user,
   - wherein the operation control system comprises a plurality of training functionalities, the training functionalities comprising:
      - a presentation functionality which is configured to cause the electronic control unit to issue a presentation command in order to present a list of noncritical contents via a user interface for the second user to indicate to the second user contents the first user is able to process.

Next, the training arrangement is specified.

According to at least one embodiment, the training arrangement comprises the training system according to any one of the embodiments specified herein. Particularly, this means that all features disclosed in connection with the training system are also disclosed for the training arrangement.

According to at least one embodiment, the training arrangement comprises a first user interface configured to receive an input of a first user and/or to present a feature to the first user. The first user interface may be part of a first electronic device, like a PC or tablet or smartphone or a notebook or laptop.

According to at least one embodiment, the training arrangement comprises a second user interface configured to receive an input of a second user and/or to present a feature to the second user. The second user interface may be part of a second electronic device, like a PC or tablet or smartphone or a notebook or laptop.

According to at least one embodiment, the first and the second user interface are operatively coupled via a wireless connection. Alternatively, the first and the second user interface may be operatively coupled via a wire connection. The first and the second user interface may be located in different rooms and/or may be spaced from each other. Each of the interfaces may be assigned its own training system. E.g. a first and a second electronic device, like a smartphone or a tablet, may each comprise both, a training system and a user interface. The training systems of the two electronic devices may then be configured to communicate with each other (wireless or via a wire) in order to perform the training.

Next, the training of the first user is specified. The training may be a therapeutic training, e.g. to treat aphasia, or a non-therapeutic training, e.g. to improve skills. The training may be a language training. The training is a method in which skills are trained.

The training may particularly be performed with the training system and/or the training arrangement specified herein. Thus, all features disclosed for the training system and/or the training arrangement are also disclosed for the training and vice versa. In particularly, some or all training functionalities of the training system may be executed or used during the training. For example, the operation control system is configured to perform the different modules specified in the following.

According to at least one embodiment, the training comprises a step in which user capability data are generated. The user capability data are indicative for the capability of the first user in content processing.

According to at least one embodiment, the training comprises a step in which a presentation command is issued depending on the user capability data in order to present a feature via a user interface. The feature is preferably related to the capability of the first user in content processing.

According to at least one embodiment, the training comprises a step, in which an individualized training module is executed. Execution of the individualized training module is also referred as individualized training session of the training, in which the first user preferably only interacts with an electronic device without a further user or person participating. During the individualized training module/session, any one of the mentioned training functionalities may be executed.

According to at least one embodiment, in the individualized training module, a presentation command is issued in order to present a task to be performed by the first user via a user interface. Synchronized to the presentation of the task, a stimulation command to a brain stimulation device may be issued or an electrical brain stimulation procedure may be performed.

According to at least one embodiment, in the individualized training module, task performance data are determined which are indicative for the performance of the first user in solving the task.

According to at least one embodiment, the method comprises a step in which a social interaction module is executed. Execution of the social interaction module is also referred as social interaction session of the training, in which the first user interacts, particularly communicates, with a second user, preferably via the training arrangement and/or the training system. Thus, the training system or training arrangement may also be referred to as communications system or communication arrangement.

The social interaction module is preferably executed after at least one individualized training session has been executed. Between the social interaction module/session and the individualized training module/session, the first user may make a break. The social interaction module can be executed time independently of the individualized training module, e.g. at any time after executing the individualized training module. During the social interaction session, any one of the mentioned training functionalities may be executed.

According to at least one embodiment, in the social interaction module, the first user and a second user interact with each other, e.g. perform a conversation. The first user and the second user are preferably not in the same room so that the interaction has to be transmitted via at least one electronic device. For example, each of the users uses a PC or a smart phone or a tablet.

According to at least one embodiment, in the social interaction module, a content, e.g. an expression, e.g. a critical or noncritical content or expression, is recognized depending on user input data provided with help of a user interface and being indicative for an input of one of the users, e.g. of the second user. The user interface may be an audio user interface, the input data may be audio user data, the input may be a speech input. The user interface may comprise a microphone. Noncritical content may be content which has been trained prior to performing the social interaction module, e.g. during an individualized training session.

The social interaction module may use as input:
- content treated in the individualized training module with data on first user's/patient's levels on content processing, e.g. expressions, word, phrases, notions information, facts etc. the first user is able to process,
- the cognitive state of the first user as outputted from a cognitive module, described below.

According to at least one embodiment, in the social interaction module, a presentation command is issued depending on the recognized expression in order to present a feature via a user interface, e.g. via the second user interface. The feature may be configured to influence the interaction between the users. E.g. the feature may be an assist feature configured to assist the first user in processing a recognized critical content. The assist feature may be presented on the second user interface to indicate the second user that a critical content has been used and that he/she should select another content. Additionally or alternatively, the assist feature may be presented on the second user interface to indicate the first user, e.g. a synonym or a graphical depiction of the critical content.

According to at least one embodiment, the method comprises a step in which a cognitive module is executed. The cognitive module may be a session or a part of a session of the training. The cognitive module may be executed during the individualized training module/session and/or during the social interaction module/session.

According to at least one embodiment, in the cognitive module, a cognitive load of the first user is determined depending on the task performance data and/or depending on psychophysiological data. The psychophysiological data are indicative for at least one psychophysiological measurement performed on the first user using a detector.

According to at least one embodiment, the method comprises a step in which the training is controlled depending on the determined cognitive load of the first user. For example, the difficulty of a task presented in the individualized training module is adapted depending on the cognitive load. Additionally or alternatively, a presentation command may be issued during the social interaction module in order to present the second user the information of the cognitive load of the first user via the second user interface.

All the steps of the training may be performed by the training system and/or by the training arrangement. Thus, the training is, in particular, a computer implemented training.

Moreover, a computer program product is specified. The computer program product comprises machine-readable instructions, which, when loaded and executed on a processor or training device or training arrangement, are configured to cause the processor or training device or training arrangement to execute the training specified herein.

Considering different user/patient needs and abilities, as well as recovery process, the training system described herein provides, inter alia, an extended speech-language platform, optionally enriched by cognitive exercises and well-being exercises. The training leads particularly to a social interaction module, which is designed in a special manner to consolidate therapy effects and/or support communication with patient's friends and family members. The training comprises more interactive, engaging and individually tailored content and flow of interaction. Additionally, it provides social interaction elements, which was proven to be beneficial in treatment of patients with aphasia and cognitive disorders, i.e. a group of patients that are socially isolated due to their inability to effectively communicate with healthy people. One of the benefits of this training is to analyze learning progress, emotional state, behavioral data (reaction time) and psychophysiological measures (e.g. cognitive load) to optimize level of task difficulty. The intended platform's informal environment is optimized to help a user in the restoration of language and cognitive skills by learning in playful, but still structured way. The consolidation of new skills in familiar and system supported environment of the social interaction module enhances successful learning. The training is suitable for various cognitive impairments that may appear due to neurodegeneration, e.g. Mild Cognitive Impairment, Alzheimer's disease, Parkinson's disease.

The social interaction module may be optimized to consolidate content learned in the individualized training module. There is strong association between tasks and exercises performed in the individualized training module and the cognitive module and the social interaction module resulting in extended environment context approach indispensable to perform real-life activities. It was proven that maintaining existing friendships and relations is beneficial, e.g. to patient recovery. Social interaction training is relevant to facilitate the return to real-life relations/to regain communication skills crucial in everyday life. The presented training may help to avoid social isolation, e.g. in aphasia.

Hereinafter, the training system, the training arrangement and the training will be explained in more detail with reference to drawings on the basis of exemplary embodiments. Same reference signs indicate same elements in the individual figures. However, the size ratios involved are not necessarily to scale, individual elements may rather be illustrated with an exaggerated size for a better understanding.

In the figures:
- Figure 1: shows a first exemplary embodiment of the training arrangement,
- Figure 2: shows a second exemplary embodiment of the training arrangement,
- Figure 3: shows an exemplary embodiment of the operation control system,
- Figure 4 to 8: show an exemplary embodiment of the training on the basis of a process diagrams,
- Figure 9: shows an exemplary embodiment of the interaction between different modules of the training,
- Figures 10 and 11: show exemplary embodiments of user interfaces,
- Figure 12: shows an exemplary embodiment of a brain stimulation device.

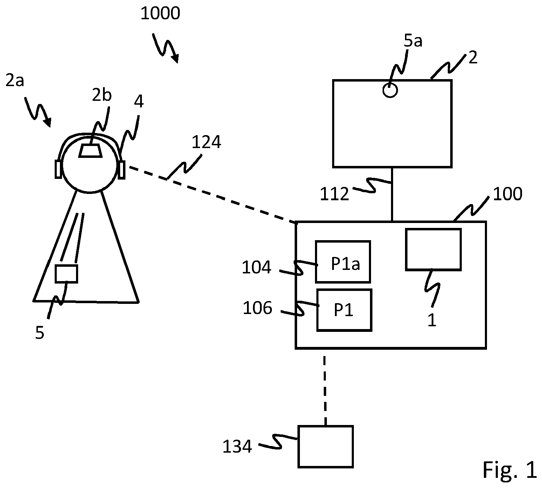

Figure 1 illustrates a first exemplary embodiment of a training arrangement 1000 which comprises:
- a training system 100,
- a first user interface 2, for instance a monitor or a touch screen, e.g. in combination with a microphone and a loudspeaker, for visible and/or audible input and output,
- a brain stimulation device 4,
- a detector 5, 5a or condition monitoring device 5, 5a, and
- a remote location device 134, e.g. at a location that is in a different room and/or visually separated from the room with the user 2a, and/or at a location that has a distance from training system 100 of more than 1 km (kilometer) or more than 10 km. The distance may be smaller than 1000 km or any other distance between two locations on planet earth.

The training system 100 and the first user interface 2 may be part of an electronic device, like a tablet, a laptop, a mobile phone, a smartphone, a personal computer, a workstation or some other kind of computing system.

The training system 100 comprises:
- a volatile memory 104, for instance RAM (Random Access Memory),
- a non-volatile memory 106,
- an electronic control unit 1, for instance CPU (Central Processing Unit) or MCU (Microcontroller Unit), and
- further parts that are not shown in detail, e.g. power source.

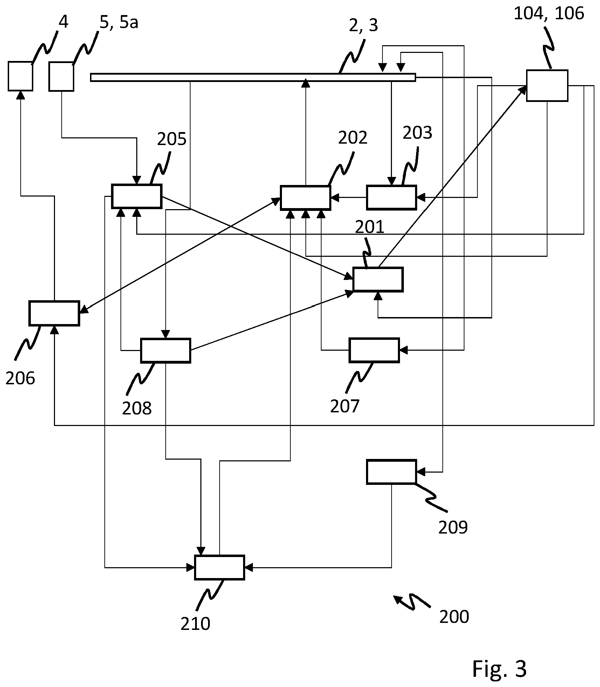

The non-volatile memory 106 stores a program code P1. The program code P1 comprises a program that realizes training functionalities of the training system 100. These functionalities are described in more detail below with regard to Figure 3. The non-volatile memory 106 may be a SSD (Solid State Disc) memory, a magnetically storing hard disk or some other kind of memory. If the training system 100 is switched on, program code P1 is copied as program code P1a and then loaded into volatile memory 104 that allows much faster reading and writing of data than non-volatile memory 106. The electronic control unit 1 executes the program code P1a and realizes an operation control system with the training functionalities. Alternatively to a non-volatile memory, the program code P1 may be supplied to the electronic control unit 1 via a data stream.

The first user interface 2 may be fixedly connected to the training system 100, as in a smartphone, mobile phone, PC (personal computer), work station, tablet, or notebook, or it may be a separate device. If the first user interface 2 is a separate device, there may be a wire connection 112 or a wireless connection between training system 100 and the first user interface 2. Furthermore, a keyboard, a computer mouse or other kinds of input and/or output devices may be part of the first user interface 2, for instance a microphone and a loudspeaker. Alternatively or additionally, earphones and/or a microphone may be comprised by or operatively connectable to the brain stimulation device 4 or the first user interface 2 for speech input and/or output.

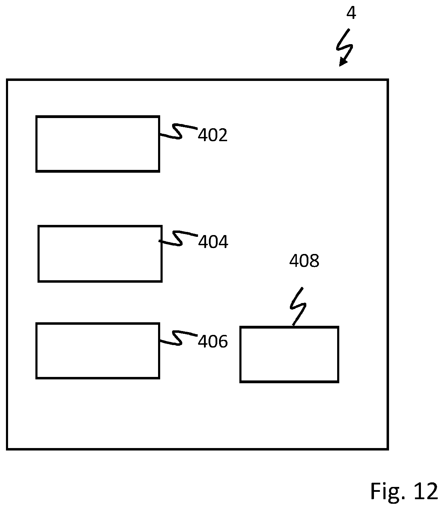

A first user 2a or a patient 2a, e.g. suffering from e.g. aphasia or a cognitive disorder, wears the brain stimulation device 4 on his/her head to enable electrical stimulation of the brain 2b. The brain stimulation device 4 may be operatively connectable or connected to the training system 100, e.g. by a wired or by wireless connection 124. Details of the brain stimulation device 4 are shown in Figure 12 and are described below.

The user condition monitor device 5 may be a device that may have the form of bracelet or a watch and/or that may be carried around the wrist. A further condition monitor device 5a, e.g. an eye tracker or a web cam, are integrated into the first user interface 2. The condition monitoring device 5 may comprise a device that measures EDA, i.e. Electro Dermal Activity, of the skin of first user 2a. Alternatively or additionally, the condition monitoring device 5 may include a device for measuring the pulse of the first user 2a that corresponds to the heart beat of the first user 2a. Alternatively or additionally, the heart rate variability (HRV) may also be detected or measured. Alternatively or additionally, voice of the first user may be recorded for analysis of user's condition. Functionalities of the training system 100 may use measurement data of the user condition monitor device 5, 5a, in particular related to the psychophysiological data described above, which may give insight into the current cognitive state/load of the first user 2a. This is explained in more detail below. There may be a wire connection or a wireless connection between the condition monitoring device 5 and the brain stimulation device 4 and/or the training system 100.

The remote location device 134 may be used by a person to get remote access to the training system 100. The remote location device 134 may be a smartphone, a tablet, a laptop, a personal computer etc. There may be a connection between the training system 100 and the remote location device 134 that uses the internet or another communication, e.g. data packet, network. The remote location device 134 may be used for the realization of functionalities of the training system 100.

The training arrangement 1000 of figure 1 may be used for executing the individualized training module/session described in more detail below.

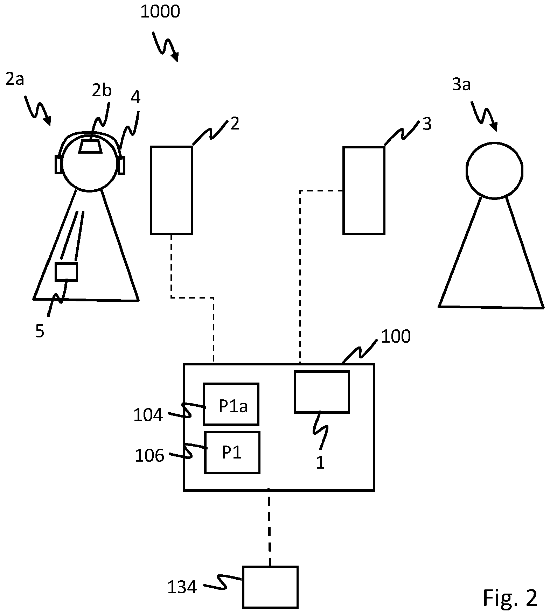

Figure 2 illustrates a second exemplary embodiment of the training arrangement 1000. This training arrangement 1000 may be used to execute the social interaction module/session described in more detail further below.

In difference to the first exemplary embodiment of the training arrangement, the training arrangement 1000 according to the second exemplary embodiment comprises a second user interface 3. The second user interface 3 may be a monitor or a touch screen e.g. in combination with a microphone and/or a loud speaker, for visible and/or audible input and output. The second user interface 3 may be part of a smart phone or mobile phone or tablet or laptop or personal computer. A second user 3a uses the second user interface for giving an input or receiving an output. The interfaces 2, 3 may be operatively coupled via wireless connection, e.g. via the internet, or via a wire connection.

In difference to what is shown in figure 2, the first user 2a may not wear the brain stimulation device 4 during the social interaction session.

Figure 3 illustrates an exemplary embodiment of the operation control system 200. The operation control system 200 comprises a plurality of training functionalities, namely:
- a data generation functionality 201,
- a presentation functionality 202,
- a recognition functionality 203,
- a cognitive load functionality 205,
- a stimulation functionality 206,
- a environment functionality 207,
- a performance assessment functionality 208,
- a well-being functionality 209,
- a master functionality 210.

### Data generation functionality 201

The data generation functionality 201 is configured to produce user capability data. The user capability data are indicative for the capability of the first user in content processing, e.g. language production and/or language comprehension. Additionally or alternatively, the content processing may concern the cognitive domain, e.g. memory skills, reasoning skills, planning skills, visuospatial functions, executive functions. The capability data may be indicative for the psychological condition, e.g. history/current state of depression, depressive episodes, psychological resources. Particularly, the user capability data may be pretreatment patient condition, i.e. information on the type of disorder of the first user. The patient information may include, e.g., type and location of a brain damage, fMRI data, list of content for treatment. Additionally, the user capability data may comprise contents, particularly expressions, i.e. words or phrases, used for the training of the first user. For example, the contents may comprise critical contents, which are contents known to be difficult for the first user to process. Additionally or alternatively, the contents may comprise noncritical contents, which are contents known to be processible by the first user.

The data generation functionality 201 may be configured to cause the electronic control unit 1 to write new user capability data in the non-volatile memory 106 or the volatile memory 104. Other training functionalities of the operation control system 200 may have access to the memories 104, 106, i.e. they may cause the electronic control unit 1 to extract and provide data from the memory 104, 106.

As can be seen in figure 3, some of the functionalities are connected to the data generation functionality 201. In this exemplary embodiment, the cognitive load functionality 205 and the performance assessment functionality 208 are connected to the data generation functionality 201. They may send cognitive load data or performance assessment data, respectively, to the data generation functionality 201 in order to store them in the memory 104, 106.

Furthermore, it can be seen in figure 3 that also the interface 2, 3 is connected to the data generation functionality 201. User input via the user interface 2, 3, e.g. feedback or contents which should be trained in future training sessions, could be inputted via the user interface 2, 3 and can then be stored in the memory 104, 106 with help of the data generation functionality 201.

### Presentation functionality 202

The presentation functionality 202 is configured to cause the electronic control unit 1 to issue presentation commands in order to present features via the interface 2, 3. The feature presented on the user interface 2, 3 may be a visible or audible feature. For example, the feature is a graphical depiction or an audible or visible (e.g. in written form) synonym of a critical content.

The presentation functionality 202 is particularly configured to cause the electronic control unit 1 to issue presentation commands in order to present tasks, e.g. training tasks and/or cognitive tasks, via the user interface 2, 3. The presentation functionality 202 is further configured to cause the electronic control unit 1 to issue other presentation commands in order to present, e.g., an environment feature on a user interface 2, 3.

The presentation functionality 202 has access to the memory 104, 106. Particularly, a presentation command may be issued depending on the user capability data stored in the memory 104, 106.

The presentation functionality 202 is connected to a master functionality 210. The master functionality 210 may communicate with the presentation functionality 202 in order to influence the presentation commands issued by the presentation functionality 202.

The presentation functionality 202 is further connected to an environment functionality 207. The environment functionality 207 may communicate to the presentation functionality 202 in order to influence a presentation command issued by the presentation functionality 202.

Moreover, the presentation functionality 202 is connected to a recognition functionality 203. In this way, the recognition functionality 203 can influence the presentation command issued by the presentation functionality 202.

### Recognition functionality 203

The recognition functionality 203 is connected to the user interface 2, 3 and receives user input data provided with help of the user interface 2, 3. The user input data are indicative for an input of a user, e.g. a speech input. The recognition functionality 203 is configured to recognize a content, e.g. a critical content, depending on the user input data, particularly to recognize the content in real time.

Recognition functionality 203 is connected to the memory 104, 106. The recognition functionality 203 may extract one content, e.g. expression, after the other from the user input data and may compare them to critical contents, e.g. critical expressions, stored in the memory 104, 106 in order to recognize a critical content in the user input data. Alternatively, the recognition functionality 203 may recognize a critical content depending on the user input data by performing a semantic analysis of the user input data. In this case, the recognition functionality 203 may not rely on the user capability data stored in the memory 104, 106.

The recognition functionality 203 is connected to the presentation functionality 202. If the recognition functionality 203 has recognized a critical content, it may communicate to the presentation functionality 202 to issue a presentation command depending on the recognized critical content in order to present an assist feature via the user interface 2, 3. The assist feature is configured to assist the first user in processing the critical content. The assist feature may be presented on the first user interface 2 or on the second user interface 3. If presented on the first user interface 2, the assist feature may be a graphical depiction or a synonym of the critical content. If the assist feature is presented on the second user interface, the assist feature may be an alert indicating that a critical content has been used and that the first user is not able to process this critical content, e.g. is not able to give a correct answer.

### Cognitive load functionality 205

The cognitive load functionality 205 is configured to determine cognitive load data depending on task performance data and/or depending on psychophysiological data and/or on self-report provide by the first user. The cognitive load data are indicative for the cognitive state of the first user.

The cognitive load functionality 205 is operatively connected to the condition monitoring devices 5, 5a. It receives psychophysiological data generated with help of the condition monitoring devices 5, 5a. Moreover, the cognitive load functionality 205 is operatively connected to the performance assessment functionality 208 and may receive performance assessment data generated with help of the performance assessment functionality 208.

The cognitive load functionality 205 may determine the cognitive load data by using an AI (Artificial Intelligence) classifier. The classifier may be loaded from the memory 104, 106. Therefore, the cognitive load functionality 205 is connected to the memory 104, 106.

The cognitive load functionality 205 is operatively connected to the data generation functionality 201. The determined cognitive load data may be communicated to the data generation functionality 201 which assures that these data are written to the memory 104, 106. The cognitive load functionality 205 is also connected to the master functionality 210. The master functionality 210 may, depending on the cognitive state of the first user, make the presentation functionality 202 to issue an appropriate presentation command. For example, the difficulty of tasks presented with help of the presentation functionality 202 may be adapted depending on the cognitive state.

### Stimulation functionality 206

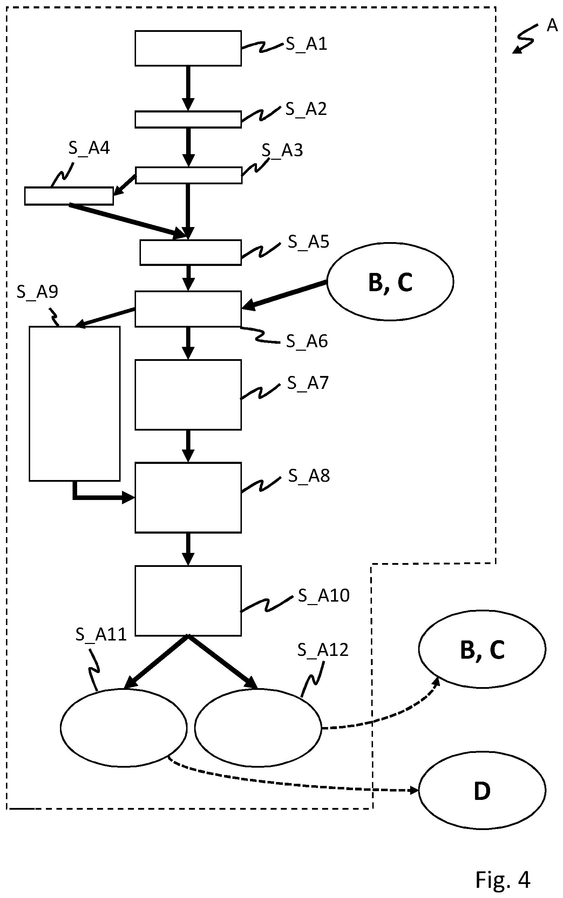

The stimulation functionality 206 may be configured to cause the electronic control unit 1 to issue a stimulation command to the electrical brain stimulation device 4 to cause the electrical brain stimulation device 4 to perform an electrical brain stimulation procedure. The electrical brain stimulation procedure is described in more detail below with reference to Figure 4.

The stimulation functionality 206 may comprise a preparation stimulation functionality for a preparation stimulation and/or a task stimulation functionality for a task stimulation. The preparation stimulation may be configured to be performed, preferably completely, before the first task of a session is presented to the first user 2a. Alternatively or additionally, the task stimulation may be configured to be synchronized with the presentation of the task. The task stimulation may be performed during the presentation of the task to the first user 2a and/or during the performance of the task by the first user 2a. For this purpose, the stimulation functionality 202 is operatively coupled to the presentation functionality 202.

The type of stimulation may be contained in the stimulation command to be transcranial electrical stimulation in constant current mode (transcranial direct current stimulation (tDCS)) or in varying current mode (e.g. transcranial alternating current stimulation (tACS), transcranial random noise stimulation (tRNS), pulses of current) or a sequence thereof.

The operation control system 200 may be configured such that during the training or a training session at least one stimulation command for varying current mode is issued. This stimulation command may be synchronized with the presentation of a task on the user interface 2. The stimulation command may be a command for a stimulation in the alternating current mode (tACS). The current in alternating current mode may have the same or a similar frequency that is also present in the natural oscillation of neurons in the relevant brain areas, e.g. brain areas that are relevant for language processing and/or production, brain areas related to memory, planning, reasoning or executive functions, as well as brain areas crucial for emotion regulation and mental well-being.

The stimulation command may include stimulation data. Alternatively or additionally, the stimulation command may refer to stimulation data that is stored in memory 104, 106. For this purpose, the stimulation functionality 206 has access to the memory 104, 106.

### Environment functionality 207

The environment functionality 207 is configured to receive environment user input data indicative for a user selected virtual environment. For this purpose, the environment functionality 207 is operatively connected to the user interface 2, 3, via which the associated user 2a, 3a may select a virtual environment for the training.

The environment functionality 207 is operatively connected to the presentation functionality 202. The presentation functionality 202 is configured to cause the electronic control unit 1 to issue a presentation command in order to present an environment feature on a user interface 2, 3, e.g. on the first user interface 2 assigned to the first user 2a. The environment feature is indicative for the user selected virtual environment.

### Performance assessment functionality 208

The performance assessment functionality 208 may be configured to calculate or to provide task performance data which is indicative for the performance of the first user 2a in performing a task, e.g. for the quality of the performance. In other words, the performance assessment functionality 208 may gather data. The task may be a training task and/or a cognitive task.

The performance assessment functionality 208 may consider at least one, an arbitrarily selected plurality of, or all of the following factors:
- 1) accuracy of performance of the task,,
- 2) duration of performance of the task,
- 3) score from last assessments or results of standard tests, e.g. feeds from standard scales (for instance feed from Western Aphasia Battery or Progressive Aphasia Severity Scale, The Mini Mental State Examination or the Montreal Cognitive Assessment),
- 4) stimulation intensity factor during performance, this factor may take into account one, and arbitrarily selected plurality of or all of: presence or absence of preparation stimulation (see Figure 4), presence or absence of task stimulation (see Figure 4), as well as optionally intensity of these stimulations or other parameters indicative for the electrical stimulations,
- 5) level of the task, there may be for instance 100 levels on a scale of 0 to 100. Scale 0 may be the easiest level.

The performance may be given a mark, e.g. on a scale of 0 to 100. The mark may be indicative for the quality, e.g. the accuracy, of the performance. The mark may take into account the number of mistakes, for instance the selection of incorrect words, number of typographical errors etc. The mark may be given manually by a supervisor, e.g. the practitioner or another person, or it may be assessed automatically, e.g. computationally, by the system 100. The duration of performance considers how long it has taken for the first user to perform the single task. The duration may be determined or measured automatically by the system 100, e.g. by measuring the time between the presentation command and a task completed signal or command which may either be generated automatically by the system 100 or require an input, e.g. by the user, the supervisor or the practitioner. Marks may be given according to ranges of duration that are predefined for the specific task. In case of written task solutions, the time between the start of the task and the end of writing may be used. The duration may also be used as an indication of a correct answer. The duration may express how easily patient executes a specific task. Each task may have a reference time, for instance based on a mean or average time which a healthy subject requires to perform the task. If the duration time is comparable to the average time it may be possible to select a more difficult task next time.

Further factors may be considered for performance assessment as well. A final score may be calculated for each task or for a part of the tasks based on one, more or all of these factors. The performance assessment functionality 208 may calculate the following after a training session:
- a medium or average score across all tasks of session; this may be used to give feedback of the progress of therapy, especially for the therapist or as motivation for first user 2a, and/or
- a medium or average score for each type of task; this may be used for detailed reports and/or for adjusting stimulation for different kinds of tasks.

The relevant tasks for performance assessment may be primarily the tasks that require activity of treated brain regions.

The performance assessment functionality 208 is operatively coupled to the cognitive load functionality 205 in order to transmit task performance data to the cognitive load functionality 205. The presentation functionality 208 is operatively coupled to the interface 2, 3 in order to receive user input data which represent the answer to the task performed by the first user 2a. The performance assessment functionality 208 is operatively coupled to the data generation functionality 201, e.g. in order to make the data generation functionality 201 to write the task performance data to the memory 104, 106. Moreover, the performance assessment functionality 208 is operatively coupled to the master functionality 210 which may make the presentation functionality 202 to issue a presentation command depending on the task performance data. For example, in this way, the difficulty of the following tasks can be changed.

The difficulty of the following tasks may be changed depending on the determined cognitive load data or the cognitive state of the first user, respectively. Information/data from the well-being functionality 209 indicative for a user input at the beginning or in the middle of a session may also be used to adapt the difficulty. That information may concern user's state, e.g. level of motivation, resilience, mood or energy.

For example, if the first user declared poor level of energy, the system 100 may detect the need for intervention earlier, it may change the level of psychophysiological pattern at which the intervention is introduced (e.g. -0,5 SD (Standard Deviation)), or the level of psychophysiological pattern from same/similar cognitive states should be introduced by the system (i.e. psychophysiological pattern that was collected when user declared the same/similar state).

After the system introduces changes to the task difficulty and flow of interaction (intervention), it collects data on user's performance and user's feedback. On the basis of collected data, the system 100 may correct the intervention in the next iteration in a more fitted way (e.g. by add or subtract 0.5, 1 or 2 SD of psychophysiological pattern / pick value and/or by changing intervention strategy by proposing different activity (e.g. breathing exercise form well-being functionality, and/or propose change of a topic of following tasks, change complexity level of the following task).

### Well-being functionality 209

The well-being functionality 209 is operatively coupled to the user interface 2, 3. The user, particularly the first user 2a, may provide feedback on his/her level of motivation, resilience, mood or energy via the user interface 2, 3, which is transformed to user input data. User input data are transmitted to the well-being module 209. The well-being module 209 may be configured to generate feedback data, which are indicative for the user feedback.

For determining the feedback data, the well-being module 209 may also be coupled to the performance assessment functionality 208 and/or the cognitive load functionality 205 (the coupling is not shown in figure 3 in order to increase the clarity of figure 3). The feedback data may be determined depending on the task performance data and/or the cognitive load data.

The feedback data may be transmitted to the master functionality 210. The master functionality 210 may then make the presentation functionality 202 to issue a presentation command depending on the feedback data in order to present a well-being feature or content via the user interface 2, 3. The well-being content may be a breathing task, a motivation task, a relaxation task, a mindfulness task, a resilience task, a shared stories task. In this way, the first user may recover and may therefore have more energy and cognitive capabilities for the next tasks. Cognitive overload may be prevented in this way.

Thus, the well-being functionality may be used to establish workload - relax balance. The well-being functionality improves usability and flexibility of the training by actively reacting to user's state. Furthermore, user condition monitoring may be used to monitor the cognitive state and emotional state of the user in order to ensure the best results of the training and prevent cognitive overloads, thus facilitate maintaining attention. Attention may be maintained longer if there are intermediate phases, e.g. of a fixed or adjustable duration, with no presentation of tasks and with no electrical stimulation procedures, preferably between two succeeding presentation commands. Whether an intermediate phase is applied between two succeeding tasks may be decided dependent on the cognitive load data.

### Master functionality 210

The master functionality 210 may coordinate or influence the presentation functionality 202 in order to present features via the user interface 2, 3 which consider the determined cognitive load data and/or the determined performance assessment data and/or the feedback data. Therefore, the master functionality 210 is operatively coupled to the cognitive load functionality 205, the performance assessment functionality 208 and the well-being functionality 209.

The operation control system 200 may also comprise a content-wizard functionality (not shown) configured such that a user, e.g. a patient, therapist or other person, may design new tasks or update existing tasks with personalized content, e.g. family photo or texts related to hobbies of a patient. Personalized, familiar content and/or interaction in environment related to chosen theme may facilitate may increase engagement, thus facilitate training outcomes.

Figures 4 to 8 illustrate an exemplary embodiment of a training. In Figure 4, an individualized training module A or individualized training session A is executed. For the individualized training session A, the first user 2a may only interact with an electronic device (PC, smartphone, laptop, tablet or notebook) without a further user being participating. For executing the individualized training session A, the arrangement 1000 of figure 1 might be used. The individualized training session A may be executed, e.g., by the operation control system 200 of figure 3.

The individualized training session A starts in step S_A1, e.g. by the first user 2a pressing a button or by selecting a button symbol on the first user interface 2. This may initiate the individualized training session A. When initiated, the first user 2a may have the brain stimulation device 4 mounted to his head and, optionally, the user condition monitor device(s) 5, 5a may be switched on.

In step S_A2, data, e.g. user capability data, are loaded from the memory 104, 106.

The next method step S_A3 checks whether a preparation stimulation has to be performed. A preparation stimulation may be necessary for instance at the beginning of the session or for specific users or patients. A question box may be shown on the first user interface 2 or data that is stored in memory 104 or 106 may be used to decide whether a preparation stimulation has to be performed or not. Whether or not the preparation stimulation is performed may be pre-set by the supervisor of the session, e.g. the practitioner/therapist or another person, and may be stored in the user capability data.

Method step S_A4 follows after method step S_A3 if a preparation stimulation should be performed. A preparation stimulation is made in method step S_A4. The preparation stimulation may be a stimulation that uses a constant stimulation current (tDCS) for brain 2b. However, other ways of stimulation are possible as well, e.g. varying current stimulation, e.g. alternating current stimulation (tACS) or pulsed current or random noise stimulation (tRNS) is also possible during preparation stimulation in addition (preferably applied in sequence, i.e. not at the same time) or instead of constant current stimulation. Preparation stimulation may have excitatory effect on stimulated areas, i.e. it may be used to raise the activity of neurons.

After the end of preparation stimulation, the success of the preparation stimulation may be reported, e.g. on the first user interface 2 or in a log file that is stored in memory 104, 106.

Method step S_A5 follows after step S_A4 if a preparation stimulation is performed and follows after step S_A3 if no preparation stimulation is performed. In step S_A5 it is checked whether a main stimulation has to be performed, i.e. a stimulation during the performance of tasks. A question box may be shown on the user interface 2 or session data stored in memory 104, 106 may be used to decide whether a main stimulation has to be performed.

If it is decided that no main stimulation has to be performed, a method step S_A6 follows after method step S_A5. In figure S_A6 a new task or a new task group (plurality of tasks), e.g. training tasks and/or cognitive tasks, is configured. The selection of the task / task group may be done manually by a practitioner or automatically based on the user capability data, e.g. data that has been approved or was input by a practitioner.

A task is presented on the first user interface in step S_A7. For example, the first user 2a is asked to actively produce the response to the task using speech or writing or to provide response to a task via selecting an answer via the first user interface 2.

In step S_A8, the response of the first user 2a is recorded. In step S_A10, task performance data are determined based on the response of the first user 2a. In step S_A11 the task performance data is stored to the memory 104, 106. The stored task performance data may then be used for executing a social interaction module D / social interaction session D explained further below. In step S_A12 the task performance data are sent to a cognitive module B, C, which will be explained further below.

The steps S_A7, S_A8, S_A10, S_A11, S_A12 may be repeated for each task of the task group. Steps S_A10, S_A11 and/or S_A12 might be performed only after several or all tasks of a task group have been performed.

If it is decided that main stimulation has to be performed in method step S_A5, a task or a task group (training tasks and/or cognitive tasks) may be selected/configured in method step S_A6. The selection of the task or task group may be done manually by a practitioner or automatically based on the user capability data.

In step S_A9 a stimulation command as described above may be generated. The current stimulation may be the same for all tasks of the task group disregarding intermediate phases in which the user relaxes. Alternatively, the current stimulation may be changed during the session that is under process or that takes place at the moment for first user 2a, for instance with regard to the kind of current stimulation, with regard to amplitude, frequency, offset, phase etc., as well as with regard to the stimulated brain area. A varying current stimulation, especially an alternating current stimulation, may be preferred. The frequency of the alternating current, e.g. a sinusoidal current, may correspond to the predominant frequency of oscillations of neurons in the relevant brain area or in the relevant brain areas. Ranges of frequencies and/or for amplitudes that may be used are given in the introductory part above. It is possible to use other forms of signals as well, for instance trapezoid, rectangular, triangular, quadratic, etc. However, constant current stimulation is also possible in addition (preferably applied in sequence, i.e. not at the same time) to varying current stimulation or instead of varying current stimulation. However, varying current stimulation where the variation is adjusted to the brain rhythm may be particularly effective for assisting the brain.

Alternatively to using the same current stimulation for all tasks of the task group, the stimulation may be dependent on the specific task to be performed. In step S_A9, the specific stimulation that belongs to the specific task is selected, e.g. using specific data stored in memory 104, 106. The specific stimulation is switched on using appropriate stimulation commands. The current stimulation may be the same for the whole task. Alternatively, the current stimulation may be changed during the performance of the task, for instance with regard to the kind of current stimulation, with regard to amplitude, frequency, offset, phase etc., as well as with regard to the stimulated brain area. A varying current stimulation, especially an alternating current stimulation, may be preferred for tasks. The frequency of the alternating current, preferably a sinusoidal current, may correspond to the predominant frequency of oscillations of neurons in the relevant brain area or in the relevant brain areas. The relevant brain areas may depend on the kind of task. However, constant current stimulation is also possible during the specific task in addition (preferably applied in sequence, i.e. not at the same time) to varying current stimulation or instead of varying current stimulation. However, varying current stimulation where the variation is adjusted to the brain rhythm may be particularly effective for assisting the brain 2b to solve the tasks.

Step S_A9 may be repeated for each task, i.e. for each repetition of steps S_A7, S_A8, S_A10, S_A11 and/or S_A12, of the task group and for each task of the task group, the specific stimulation is selected. During the presentation of specific tasks, main stimulation may be paused or continued.

As can be seen in figure 4, the selection of the task or task group in step S_A6 is also dependent on an input of the cognitive module B, C, which will be explained in more detail further below.

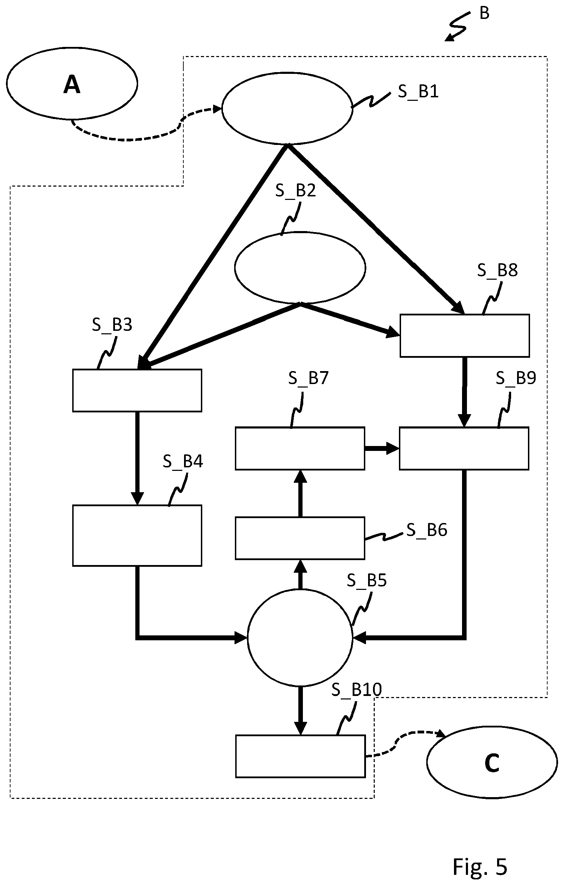

Figure 5 shows the execution of a first part B of the cognitive module B, C. The first part B may be executed in parallel to the individualized training session A, e.g. during or in between the presentation of tasks.

In step S_B1, task performance data from the individualized training module A are received. In step S_B2, psychophysiological data are generated with help of the condition monitoring device(s) 5, 5a. Additionally or alternatively, psychophysiological-related data may be self-reported by the user. In step S_B3, the task performance data and the psychophysiological data are combined and synchronized. This means that the psychophysiological data which are associated with the cognitive state of the first user when solving one or more tasks are combined with the task performance data assigned to that task or these tasks, respectively.

In step S_B4, psychophysiological load metrics or psychophysiological patterns (such as parameters for specific changes of the collected psychophysiological data) are calculated from the psychophysiological data. Performance indicators indicating the performance of the user in solving the task, e.g. score, reaction time, time for solving the task, are calculated from the task performance data. This may be called first data set. This first data set is indicative for the cognitive state of the first user.

In step S_B8, the task performance data and the psychophysiological data are added to a buffer of the training system 100.

In step S_B9, the buffered data are classified using a classifier. The classifier may be an AI (Artificial Intelligence) classifier, e.g. comprising a neural network, which gets as inputs the buffer data and which provides as an output classified data. It may be possible to use only the buffered psychophysiological data as an input for the classifier in order to determine the classified data. The classified data are indicative for the cognitive state of the first user.

In step S_B5 the first data set and the classified data are combined in order to determine cognitive load data being inactive for the cognitive state of the fist user. Using the fist data set and the classified data may increase the accuracy.

In steps S_B6 and S_B7 the classifier is updated and trained based on and with the first data set, the classified data and the determined cognitive load data of step S_B5.

In step S_B10 the cognitive state of the first user may be transmitted to a second part C of the cognitive module B, C.

The classifier may be trained to detect individual tendencies (either in psychophysiological data and/or task performance data and/or self-report) indicative of a cognitive overload before actual cognitive overload occurs.

It shall be noticed that the buffered data may comprise data from more than one task and the corresponding psychophysiological data. In this way a tendency in the data may be taken into account when determining the cognitive load with help of the classifier.

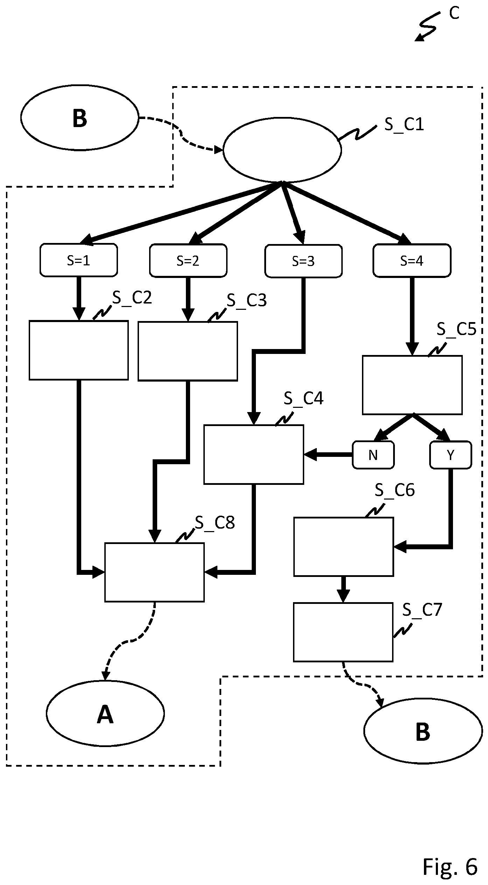

Figure 6 illustrates the execution of the second part C of the cognitive module B, C.

In step S_C1, the input from the first part B is received. The cognitive state of the first user is assigned to one of four pre-classified states. The pre-classified states, S for short, may be defined as follows:
S=1: the task performance is good and the psychophysiological load is high.
S=2: the task performance is good and the psychophysiological load is low.
S=3: the task performance is low and the psychophysiological load is low.
S=4: the task performance is low and the psychophysiological load is high.

If S=1, a step S_C2 is executed in which it is decided that the level of the tasks, i.e. the difficulty of the tasks, is good and should be kept as it is. Indeed, a high psychophysiological load compared with a high task performance may indicate that the difficulty of the tasks is optimal and effective for the training outcomes.

If S=2, a step S_C3 is executed, in which it is decided that the level of the tasks is too low and should be increased. Indeed, if the task performance is high but the psychophysiological load is low, this might indicate that the tasks are too easy and the difficulty should be increased.

If S=3, a step S_C4 is executed, in which it is decided that the type of task should be changed. Indeed, if the task performance is low and the psychophysiological load is low, it might be assumed that the task is not engaging and that the level should be increased or the topic should be changed or another type of activity should be proposed. In an optional step, the first user might be asked, which kind of tasks he would do like to do next.

If S=4, a step S_C5 may be executed, in which it is decided whether the training should be interrupted and the user should make a break to come down. Indeed, low task performance and a high psychophysiological load might indicate that the user is overloaded after intense training. The operation control system may allow the first user to perform more tasks, but may prompt user whether he/she wishes to take a break. Alternatively, if this condition is kept for longer period of time, the operation control system may trigger the break automatically. If it is decided to take a break, a feature or task might be presented on the first interface in step S_C6 which helps the user to relax. For example, a breathing exercise is proposed. In step SC_7, after step S_C6, the cognitive load/state may be checked or verified by again executing the first part B of the cognitive module B, C. For example, the cognitive state is the verified only depending on the psychophysiological data.

If it is decided not to take a break in step S_C5, the step S_C4 could be performed next in which it is decided that the type of task should be changed.

In step S_C8 the decision from steps S_C2, S_C3 or S_C4 are communicated to the individualized training session A/individualized training module A which, in step S_A6, appropriately selects the new task or new task group.

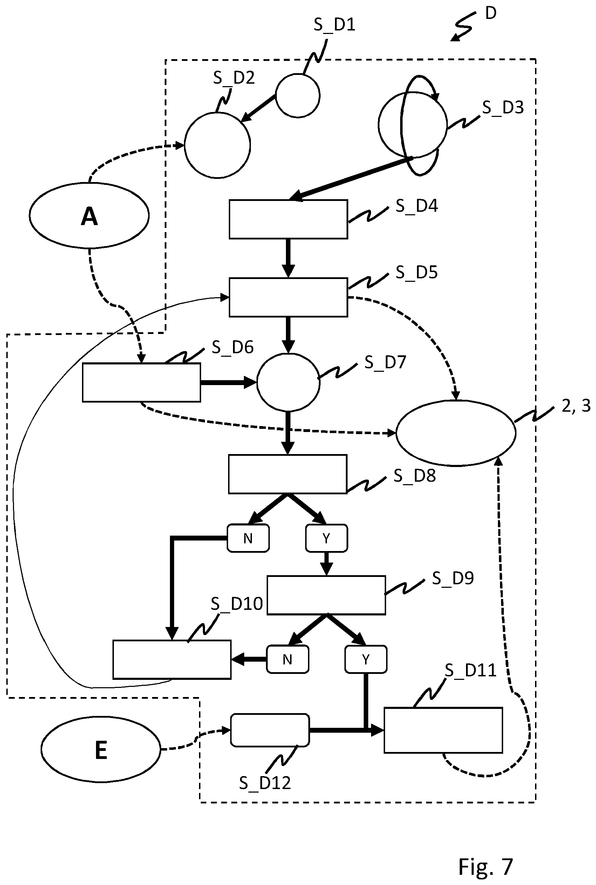

In figure 7, a social interaction module D / social interaction session D of the training is executed. For executing the social interaction session D, the training arrangement 1000 of figure 2 might be used. During the social interaction session D, the first user 2a interacts, e.g. communicates, with a second user 3a via the user interfaces 2, 3.

In a step S_D1, which is optional, the first user or the second user selects a virtual environment for the interaction. For example, the user presses a button on the respective user interface 2, 3 to select the environment. The environment is a virtual environment at which the interaction between the two users should happen. For example, the environment may be a restaurant, a picnic area, a cinema, a theatre, a walk, a gym, a photo album, a beauty salon, an intimacy/romantic area, an office. For example, a restaurant is selected as the virtual environment.

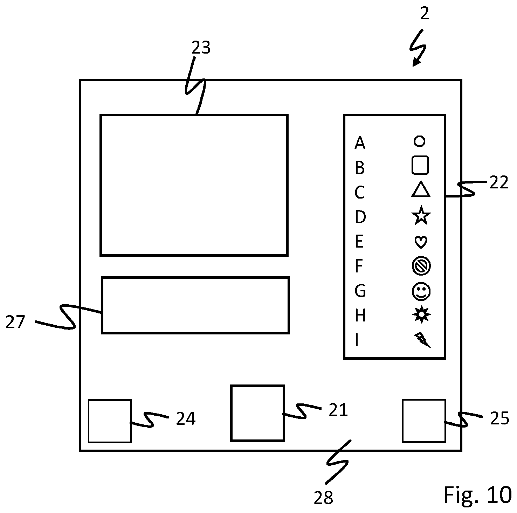

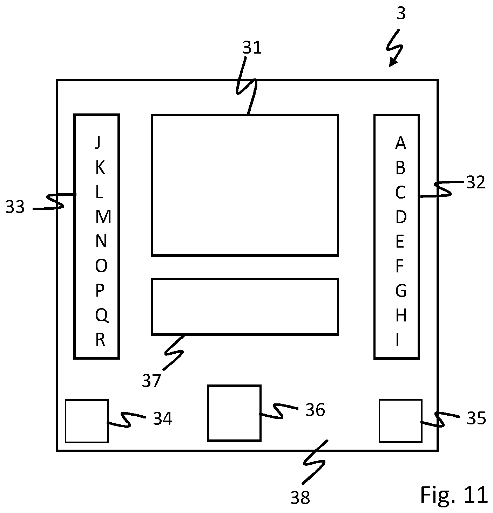

In step S_D2, a display 28 of the first user interface 2 presents a feature which is indicative for the selected virtual environment (see figure 10). For example, the selected virtual environment is indicated as a 2D restaurant, e.g. uploaded from Google Street View, and forms a display background. Likewise, a display 38 of the second user interface 3 may present such a feature being indicative for the selected virtual environment (see figure 11).

Moreover, in step SD_2, a first user dictionary 22 is presented on the second interface 2 (see figure 10). This dictionary 22 presents critical contents, e.g. critical expressions, which the first user has problems to process, e.g. comprehend and/or to pronounce. For example, the critical contents are presented by pictures and/or symbols in the dictionary 22. This allows the first user to quickly search for the critical contents he has problems to process. The dictionary 22 may be selected depending on the selected virtual environment. For example, all critical contents are selected which are related to the selected virtual environment. For example, the critical contents concern: naming of cutlery, dishes, drinks or ordering a meal etc.

Furthermore, in step S_D2, content clouds 32, 33 may be presented on the second user interface 3 assigned to the second user 3a (see figure 11). The first content cloud 32 may display noncritical contents, which the first user is able to process. The first content cloud 32 might present contents, like expressions, words and/or phrases, that should be used during the interaction, e.g. contents which have been previously treated in the individualized training module A. The second content cloud 33 might indicate critical contents, which, e.g., should be avoided in the interaction. The critical contents may also result from this individualized training module A. The content clouds 32, 33 may also be selected based on the selected virtual environment.

Using the assigned user interfaces 2, 3, the first user 2a and the second user 3a start an interaction, e.g. a conversation, in step S_D3. Each of the users 2a, 3a might therefore speak into a microphone 25, 35 of the respective user interface 2, 3 or may write or type on the respective user interface 2, 3. Each user may hear the other user via a loudspeaker 24, 34 assigned to the respective user interface 2, 3. By way of example, speech recording is performed in step S_D3.

In a step S_D4, recorded user input data of the users 2a, 3a generated with help the interfaces 2, 3 are added to a buffer.

In step S_D5, one content, e.g. expression, after the other is extracted from the buffered user input data. This is preferably performed in real-time with help of an appropriate software tool. The extracted contents may be displayed on the user interfaces 2, 3 in corresponding display areas 27, 37 (see figure 10 and 11). For example, the extracted contents may be displayed in written form.

In step S_D6, critical and non-critical contents are loaded, e.g. from the memory 104, 106. The critical and non-critical contents may have been generated or updated with help of the individualized training module A.

In step S_D7, the extracted contents of step S_D5 may be compared to the contents loaded in step S_D6. In step S_D8, it is determined whether the extracted content corresponds to a content of the data base.

If this is the case (Y), a step S_D9 is executed in which it is determined if the extracted content is a critical content.

If this is the case (Y), a step S_D11 is executed, in which, e.g., a corresponding alert is presented to one or both of the user interfaces 2, 3. For example, an alert is presented on the second user interface 3 indicating that the second user has used a critical content. A suggestion to rephrase the critical content or a suggestion for an alternative content may be presented via the second user interface 3. On the first user interface 2, a synonym of the critical content and/or an image or symbol representing the critical content may be presented in order to help the first user.

If it turns out in step S_D9 that the extracted content is not a content of the database (N), a step S_D10 is executed, in which it is decided that the next content should be investigated. Therefore, it is jumped to step S_D5 and the next content is extracted from the buffered data.

If it turns out in step S_D9 that the extracted content is a noncritical content, step S_D10 is executed.

The social interaction module D may comprise a step S_D12 in which cognitive load data or a cognitive state determined with help of a cognitive module E is received. If the cognitive load data / cognitive state indicate that the cognitive load of the first user is high, the step S_D11 is executed and an alert is presented on one or both of the user interfaces 2, 3.

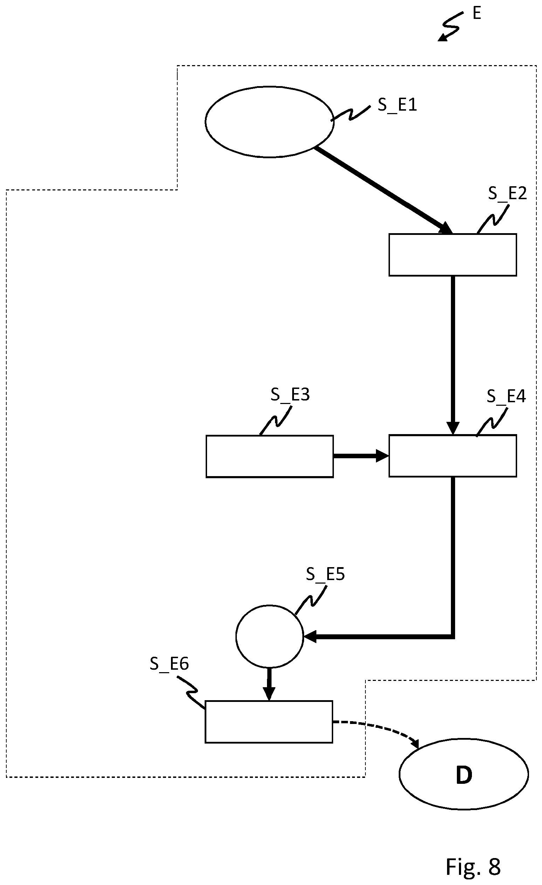

The cognitive module E is illustrated in figure 8. For performing the cognitive module E, psychophysiological data of the first user 2a are extracted and are received in step S_E1. The psychophysiological data are stored in a buffer in step S_E2. In steps S_E3 and S_E4, the buffered psychophysiological data are classified with help of a classifier, which might be the classifier describes in connection with figure 5.

In step S_E5, cognitive load data are extracted from the classified buffered data and in step S_E6 the cognitive load data or a determined cognitive state are transmitted to the social interaction module D.

As an example: the psychophysiological data are indicative for an increasing heart rate of the first user and/or an increasing pupil diameter and/or an increasing number of Skin Conductance Responses (SCR). The classifier has learned that a specific change in one or more or all of these parameters is indicative for an increased cognitive load or an upcoming cognitive load. Accordingly, cognitive load data are generated, which are indicative for the increased cognitive load or the upcoming increased cognitive load. Depending on the cognitive load data, the operation control system then controls the training of the first user.

When executing the social interaction module D, the contents extracted from the buffered data may also be used to perform a sematic analysis in order to extract the topic of the interaction or conversation. Contents related to the topic and which should be trained, e.g. critical or noncritical contents, may then be presented on one or both user interfaces 2, 3.

Semantic analysis may, e.g., be used to identify if the topic of the interaction comprises contents, e.g. expressions, notions, information or facts, that are known to be difficult to be processed by the first user, if the first user is a patient suffering from a disorder, e.g. MCI or Alzheimer's disease.

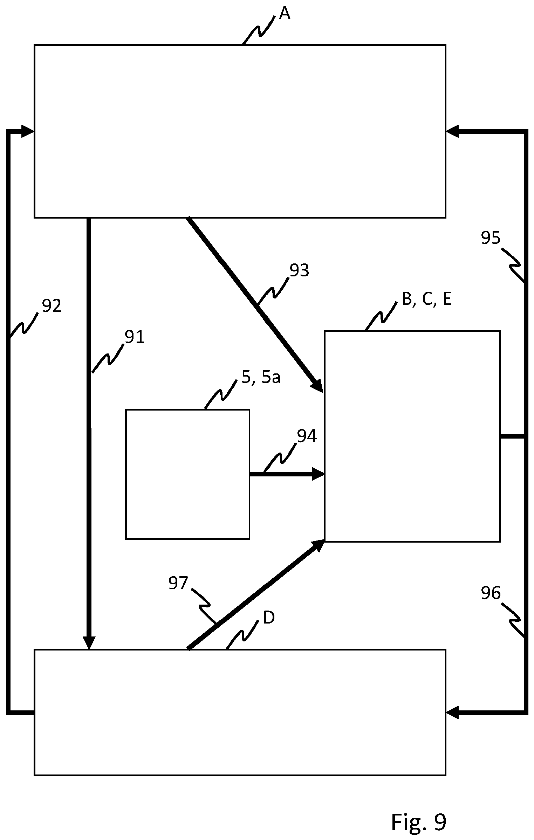

Figure 9 illustrates the interaction between the different modules of the training. The individualized training module A provides contents, e.g. noncritical or critical contents, for the social interaction module D (connection 91). The social interaction module D provides the possibility for the first user and/or the second user to select contents to be further trained and this may be provided for the individualized training module A (connection 92).

Task performance data are provided with help of the individualized training module A and these task performance data are transferred to the cognitive module B, C, E (connection 93).

Psychophysiological data of the first user 2a are provided with help of user condition monitoring devices 5, 5a and these data are also transferred to the cognitive module B, C, E (connection 94). Depending on these data, the cognitive module B, C, E determines cognitive load data and/or the cognitive state of the first user 2a. The cognitive load data are transferred to the individualized training module A or the social interaction module D (connections 95 and 96). The cognitive module B, C, E may be triggered to check the cognitive state of the first user during performing the social interaction module D (connection 97). The triggering may be done by the first and/or second user.

Figure 10 shows an exemplary embodiment of a first user interface 2 for the first user 2a. The elements 22, 24, 25, 27 and 28 have already been explained. The first user interface 2 comprises also one or more quick control buttons 21, wherein each quick control button may be used to provide a specific information, request or answer to the interlocutor (second user 3a), e.g. "Repeat, please.", "Rephrase, please.", "Please wait before continuing". These buttons may trigger the presentation of relevant information on the second user interface 3.

Display area 23 may be used to show the face of the interlocutor (second user). The display area 27 may be further used as an input field to write down or select shown expressions which should be trained in the individualized training module A.

Figure 11 shows an exemplary embodiment of the second user interface 3. The elements 32, 33, 34, 35, 37 and 38 have already been explained before. Additionally to them, there is an alert area 36, for example a blinking LED or a display area, which indicates an alert if a cognitive overload of the first user is recognized, for example with help of the cognitive module E.

The display area 31 may be used to show the face of the interlocutor (first user). The display area 37 may be further used as an input field to write down or select content which should be trained in the individualized training module A. There may be an additional content update feature on the second user interface 3 (not shown) for triggering a content upload for the individualized training module A.

Figure 12 illustrates parts of the brain stimulation device 4. The brain stimulation device 4 may comprise:
- a communication interface 402,
- a control unit 404, for instance an MCU (Microcontroller Unit) or a CPU (Control Processing Unit),
- at least one electrode 406 or at least two electrodes 406, preferably three or more electrodes, where, preferably, at least one of the electrodes 406 is configured to be arranged on the head of the first user 2a and the other electrode may be placed for instance on an appropriate location of the body of the first user 2a, for instance on his or her shoulder, and
- an electrical power supply unit 408, for instance a battery or an accumulator.

Communication interface 402 may be a wireless interface, for instance a Bluetooth interface. Stimulation commands may be received from training system 100 via communication interface 402, especially via a receiving unit of the interface (not illustrated). The interface 402 may comprise a transmitting unit. Confirmation messages may be sent from communication interface 402 to the training system 100 using the transmitting unit.

The control unit 404 may control and coordinate the processing of stimulation commands within brain stimulation device 4. The at least two electrodes 406 are connected to appropriate output circuitry. The control unit 404 sends signals or data to this output circuitry according to the stimulation commands. Thus, it is possible to generate constant current output or varying current output at electrodes 406 as desired. A gel may be used to improve current transmission between the electrodes 406 and the cranium and/or brain 2b of the first user 2a. Electrical power supply unit 408 supplies electrical energy to other units of brain stimulation device 4.

This patent application claims priority to European patent application 20461559 5.

### Reference sign list

- 1: control unit
- 2: first interface
- 2a: first user
- 2b: brain of first user
- 3: second interface
- 3a: second user
- 4: brain stimulation device
- 5: detector/ condition monitoring device
- 5a: detector/ condition monitoring device
- 21: quick control button
- 22: first user dictionary
- 23: display area
- 24: loudspeaker
- 25: microphone
- 27: display area and input field
- 28: display
- 31: display area
- 32: content cloud
- 33: content cloud
- 34: loudspeaker
- 35: microphone
- 36: alert
- 37: display area and input field
- 38: display
- 91 ... 97: connections
- 100: training system
- 104: volatile memory
- 106: nonvolatile memory
- 124: wireless connection
- 134: remote location device
- 200: operation control system
- 201: data generation functionality
- 202: presentation functionality
- 203: recognition functionality
- 205: cognitive load functionality
- 206: stimulation functionality
- 207: environment functionality
- 208: performance assessment functionality
- 209: well-being functionality
- 210: master functionality
- 402: communication interface
- 404: control unit
- 406: electrode
- 408: electric power supply
- P1: program code
- P1a: copied program code
- A: individualized training module
- B, C, E: cognitive module
- D: social interaction module
- S_A1 ... S_A12: method steps
- S_B1 ... S_B10: method steps
- S_C1 ... S_C8: method steps
- S_D1 ... S_D12: method steps
- S_E1 ... S_E6: method steps

## Claims

1. Training system (100) for a training of a first user (2a) comprising
a) a computer program product comprising program code (P1) which, when loaded and executed on an electronic control unit (1), provides an operation control system (200), and/or
b) an electronic control unit (1) with a program code (P1), wherein the program code (P1), when executed on the electronic control unit (1), provides an operation control system (200),
- wherein the operation control system (200) is configured to control the training of the first user (2a),
- wherein the operation control system (200) comprises a plurality of training functionalities, the training functionalities comprising:
- a data generation functionality (201) configured to produce user capability data, the user capability data being indicative for the capability of the first user (2a) in content processing,
wherein the content includes: language related content, memory related content, executive related content, visuospatial related content, emotional content,
wherein the content comprises critical contents the first user (2a) has problems to process, - a presentation functionality (202) which is configured to cause the electronic control unit (1) to issue a first presentation command depending on the user capability data in order to present a feature via a user interface (2, 3), the feature being related to the capability of the first user (2a) in the content processing,
wherein the presentation functionality (202) is configured to cause the electronic control unit (1) to issue the first presentation command in order to present a list of the critical contents via the user interface (2, 3) to indicate contents the first user (2a) has problems to process.

2. The training system (100) according to claim 1, wherein
- the training functionalities comprise a recognition functionality (203) which is configured to recognize a critical content depending on user input data provided with help of a user interface (2, 3) and being indicative for an input of a user (2a, 3a), wherein the critical content is a content the first user (2a) has problems to process,
- the presentation functionality (202) is configured to cause the electronic control unit (1) to issue a presentation command depending on the recognized critical content in order to present an assist feature via a user interface (2, 3), the assist feature being configured to assist the first user (2a) in processing the critical content.

3. The training system (100) according to claim 2,
- wherein the recognition functionality (203) is configured to recognize the critical content depending on user input data provided with help of a user interface (3) and being indicative for an input of a second user (3a).

4. The training system (100) according to claim 2 or 3, wherein
- the user capability data are indicative for one or more critical contents known to make problems to the first user (2a) to process,
- the recognition functionality (203) is configured to recognize the critical content depending on the user input data and the user capability data.

5. The training system (100) according to any one of the preceding claims, wherein
- the presentation functionality (202) is configured to cause the electronic control unit (1) to issue a second presentation command in order to present a list of noncritical contents via a user interface (2, 3) to indicate contents the first user (2a) is able to process, and/or
- the first presentation command and/or the second presentation command are configured to present the list of critical contents via a user interface (3) for a second user (3a)

6. The training system (100) according to any one of the preceding claims, wherein
- the presentation functionality (202) is configured to cause the electronic control unit (1) to issue a presentation command in order to present a cognitive task via a user interface (2, 3), the cognitive task being a task to be performed by the first user (2a).

7. The training system (100) according to any one of the preceding claims, wherein
- the training functionalities comprise a performance assessment functionality (208), wherein the performance assessment functionality (208) is configured to determine task performance data which is indicative for the performance of the first user (2a) in solving a task,
- the training functionalities comprise a cognitive load functionality (205) configured to determine cognitive load data depending on the task performance data and/or depending on psychophysiological data, wherein
- the cognitive load data are indicative for the cognitive load of the first user (2a),
- the psychophysiological data are indicative for at least one psychophysiological measurement performed on the first user (2a) using a detector (5, 5a),
- the operation control system (200) is configured to control the training depending on the determined cognitive load of the first user (2a).

8. The training system (100) according to claim 7, wherein
- the cognitive load functionality (205) is configured to the determine cognitive load data depending on self-report provided by the first user (2a).

9. The training system (100) according to any one of the preceding claims, wherein
- the training functionalities comprise a stimulation functionality (206) which is configured to cause the electronic control unit (1) to issue a stimulation command to an electrical brain stimulation device (4, 5) to cause the electrical brain stimulation device (4, 5) to perform an electrical brain stimulation procedure,
- the presentation functionality (202) is configured to cause the electronic control unit (1) to issue a presentation command in order to present a training task to be performed by the first user (2a) via a user interface (2, 3),
- the stimulation functionality (206) comprises a task stimulation functionality for a task stimulation,
- the task stimulation is configured to be synchronized with the presentation of the training task.

10. The training system (100) according to any one of the preceding claims, wherein
- the training functionalities comprise an environment functionality (207) which is configured to receive environment user input data indicative for a user selected virtual environment at which the first user (2a) performs the training,
- the presentation functionality (202) is configured to cause the electronic control unit (1) to issue a presentation command in order to present an environment feature on a user interface (2, 3), the environment feature being indicative for the user selected virtual environment.

11. The training system (100) according to any one of the preceding claims, wherein
- the data generation functionality (201) is configured to produce the user capability data depending on an input provided with help of a user interface (2, 3) and performed by a user (2a, 3a).

12. The training system (100) according to any one of the preceding claims, wherein the response production includes speaking and/or writing and/or indicating a preferred option among available options.

13. The training system (100) according to any one of the preceding claims, wherein the content processing comprises or is language production and/or language comprehension, and
wherein the presentation functionality (202) is configured to cause the electronic control unit (1) to issue the first presentation command in order to present a list of critical words or phrases via the user interface (2, 3) to indicate words or phrases the first user (2a) has problems to produce and/or to comprehend.

14. The training system (100) according to any one of the preceding claims, wherein the content processing includes one or more or all of: content comprehension, content acknowledgement, response production related to the content.

15. A training arrangement (1000) comprising:
- the training system (100) according to any one of the preceding claims,
- a first user interface (2) configured to receive an input of a first user (2a) and/or to present a feature to the first user (2a),
- a second user interface (3) configured to receive an input of a second user (3a) and/or to present a feature to the second user (3a).

16. The training arrangement (1000) according to claim 15, wherein
- the first (2) and the second (3) user interfaces are operatively coupled via a wireless connection or a wire connection.

## Patentansprüche

1. Trainingssystem (100) für ein Training eines ersten Benutzers (2a) umfassend
a) ein Computerprogrammprodukt, das Programmcode (P1) umfasst, der, wenn er auf einer elektronischen Steuerungseinheit (1) geladen und ausgeführt wird, ein Betriebssteuerungssystem (200) bereitstellt, und/oder
b) eine elektronische Steuerungseinheit (1) mit einem Programmcode (P1), wobei der Programmcode (P1), wenn er auf der elektronischen Steuerungseinheit (1) ausgeführt wird, ein Betriebssteuerungssystem (200) bereitstellt,
- wobei das Betriebssteuerungssystem (200) dazu konfiguriert ist, das Training des ersten Benutzers (2a) zu steuern,
- wobei das Betriebssteuerungssystem (200) eine Vielzahl von Trainingsfunktionalitäten umfasst, wobei die Trainingsfunktionalitäten Folgendes umfassen:
- eine Datengenerierungsfunktionalität (201), die dazu konfiguriert ist, Benutzerfähigkeitsdaten zu erzeugen, wobei die Benutzerfähigkeitsdaten für die Fähigkeit des ersten Benutzers (2a) bei der Inhaltsverarbeitung bezeichnend sind,
wobei der Inhalt Folgendes beinhaltet: sprachbezogener Inhalt, gedächtnisbezogener Inhalt, ausführungsbezogener Inhalt, visuell-räumlicher bezogener Inhalt, emotionaler Inhalt,
wobei der Inhalt kritische Inhalte umfasst, bei denen der erste Benutzer (2a) Probleme hat, sie zu verarbeiten,
- eine Präsentationsfunktionalität (202), die dazu konfiguriert ist, die elektronische Steuerungseinheit (1) zu veranlassen, einen ersten Präsentationsbefehl in Abhängigkeit von den Benutzerfähigkeitsdaten auszugeben, um ein Feature über eine Benutzerschnittstelle (2, 3) zu präsentieren, wobei sich das Feature auf die Fähigkeit des ersten Benutzers (2a) bei der Inhaltsverarbeitung bezieht,
wobei die Präsentationsfunktionalität (202) dazu konfiguriert ist, die elektronische Steuerungseinheit (1) zu veranlassen, den ersten Präsentationsbefehl auszugeben, um eine Liste der kritischen Inhalte über die Benutzerschnittstelle (2, 3) zu präsentieren, um Inhalte anzugeben, bei denen der erste Benutzer (2a) Probleme hat, sie zu verarbeiten.

2. Trainingssystem (100) nach Anspruch 1, wobei
- die Trainingsfunktionalitäten eine Erkennungsfunktionalität (203) umfassen, die dazu konfiguriert ist, einen kritischen Inhalt in Abhängigkeit von Benutzereingabedaten zu erkennen, die mit Hilfe einer Benutzerschnittstelle (2, 3) bereitgestellt werden und für eine Eingabe eines Benutzers (2a, 3a) bezeichnend sind, wobei der kritische Inhalt ein Inhalt ist, bei dem der erste Benutzer (2a) Probleme hat, ihn zu verarbeiten,
- die Präsentationsfunktionalität (202) dazu konfiguriert ist, die elektronische Steuerungseinheit (1) zu veranlassen, einen Präsentationsbefehl in Abhängigkeit von dem erkannten kritischen Inhalt auszugeben, um ein Unterstützungsfeature über eine Benutzerschnittstelle (2, 3) zu präsentieren, wobei das Unterstützungsfeature dazu konfiguriert ist, den ersten Benutzer (2a) bei dem Verarbeiten des kritischen Inhalts zu unterstützen.

3. Trainingssystem (100) nach Anspruch 2,
- wobei die Erkennungsfunktionalität (203) dazu konfiguriert ist, den kritischen Inhalt in Abhängigkeit von Benutzereingabedaten zu erkennen, die mit Hilfe einer Benutzerschnittstelle (3) bereitgestellt werden und für eine Eingabe eines zweiten Benutzers (3a) bezeichnend sind.

4. Trainingssystem (100) nach Anspruch 2 oder 3, wobei
- die Benutzerfähigkeitsdaten für einen oder mehrere kritische Inhalte bezeichnend sind, von denen bekannt ist, dass sie dem ersten Benutzer (2a) Probleme bereiten, sie zu verarbeiten,
- die Erkennungsfunktionalität (203) dazu konfiguriert ist, den kritischen Inhalt in Abhängigkeit von den Benutzereingabedaten und den Benutzerfähigkeitsdaten zu erkennen.

5. Trainingssystem (100) nach einem der vorhergehenden Ansprüche, wobei
- die Präsentationsfunktionalität (202) dazu konfiguriert ist, die elektronische Steuerungseinheit (1) zu veranlassen, einen zweiten Präsentationsbefehl auszugeben, um eine Liste unkritischer Inhalte über eine Benutzerschnittstelle (2, 3) zu präsentieren, um Inhalte zu bezeichnen, bei denen der erste Benutzer (2a) in der Lage ist, sie zu verarbeiten, und/oder
- der erste Präsentationsbefehl und/oder der zweite Präsentationsbefehl dazu konfiguriert sind, die Liste kritischer Inhalte über eine Benutzerschnittstelle (3) für einen zweiten Benutzer (3a) zu präsentieren.

6. Trainingssystem (100) nach einem der vorhergehenden Ansprüche, wobei
- die Präsentationsfunktionalität (202) dazu konfiguriert ist, die elektronische Steuerungseinheit (1) zu veranlassen, einen Präsentationsbefehl auszugeben, um eine kognitive Aufgabe über eine Benutzerschnittstelle (2, 3) zu präsentieren, wobei die kognitive Aufgabe eine Aufgabe ist, die von dem ersten Benutzer (2a) durchzuführen ist.

7. Trainingssystem (100) nach einem der vorhergehenden Ansprüche, wobei
- die Trainingsfunktionalitäten eine Leistungsbewertungsfunktionalität (208) umfassen, wobei die Leistungsbewertungsfunktionalität (208) dazu konfiguriert ist, Aufgabenleistungsdaten zu bestimmen, die für die Leistung des ersten Benutzers (2a) beim Lösen einer Aufgabe bezeichnend sind,
- die Trainingsfunktionalitäten eine kognitive Belastungsfunktionalität (205) umfassen, die dazu konfiguriert ist, kognitive Belastungsdaten in Abhängigkeit von den Aufgabenleistungsdaten und/oder in Abhängigkeit von psychophysiologischen Daten zu bestimmen, wobei
- die kognitiven Belastungsdaten für die kognitive Belastung des ersten Benutzers (2a) bezeichnend sind,
- die psychophysiologischen Daten für mindestens eine psychophysiologische Messung, die an dem ersten Benutzer (2a) unter Verwendung eines Detektors (5, 5a) durchgeführt wird, bezeichnend sind,
- das Betriebssteuerungssystem (200) dazu konfiguriert ist, das Training in Abhängigkeit von der bestimmten kognitiven Belastung des ersten Benutzers (2a) zu steuern.

8. Trainingssystem (100) nach Anspruch 7, wobei
- die kognitive Belastungsfunktionalität (205) dazu konfiguriert ist, kognitive Belastungsdaten in Abhängigkeit von einem Selbstbericht, der von dem ersten Benutzer (2a) bereitgestellt wird, zu bestimmen.

9. Trainingssystem (100) nach einem der vorhergehenden Ansprüche, wobei
- die Trainingsfunktionalitäten eine Stimulationsfunktionalität (206) umfassen, die dazu konfiguriert ist, die elektronische Steuerungseinheit (1) zu veranlassen, einen Stimulationsbefehl an eine elektrische Hirnstimulationsvorrichtung (4, 5) auszugeben, um die elektrische Hirnstimulationsvorrichtung (4, 5) zu veranlassen, ein elektrisches Hirnstimulationsverfahren durchzuführen,
- die Präsentationsfunktionalität (202) dazu konfiguriert ist, die elektronische Steuerungseinheit (1) zu veranlassen, einen Präsentationsbefehl auszugeben, um eine Trainingsaufgabe, die von dem ersten Benutzer (2a) durchzuführen ist, über eine Benutzerschnittstelle (2, 3) zu präsentieren,
- die Stimulationsfunktionalität (206) eine Aufgabenstimulationsfunktionalität für eine Aufgabenstimulation umfasst,
- die Aufgabenstimulation dazu konfiguriert ist, mit der Präsentation der Trainingsaufgabe synchronisiert zu werden.

10. Trainingssystem (100) nach einem der vorhergehenden Ansprüche, wobei
- die Trainingsfunktionalitäten eine Umgebungsfunktionalität (207) umfassen, die dazu konfiguriert ist, Umgebungsbenutzereingabedaten zu empfangen, die für eine vom Benutzer ausgewählte virtuelle Umgebung bezeichnend sind, in der der erste Benutzer (2a) das Training durchführt,
- die Präsentationsfunktionalität (202) dazu konfiguriert ist, die elektronische Steuerungseinheit (1) zu veranlassen, einen Präsentationsbefehl auszugeben, um ein Umgebungsfeature auf einer Benutzerschnittstelle (2, 3) zu präsentieren, wobei das Umgebungsfeature für die vom Benutzer ausgewählte virtuelle Umgebung bezeichnend ist.

11. Trainingssystem (100) nach einem der vorhergehenden Ansprüche, wobei
- die Datengenerierungsfunktionalität (201) dazu konfiguriert ist, die Benutzerfähigkeitsdaten in Abhängigkeit von einer Eingabe zu erzeugen, die mit Hilfe einer Benutzerschnittstelle (2, 3) bereitgestellt und von einem Benutzer (2a, 3a) durchgeführt wird.

12. Trainingssystem (100) nach einem der vorhergehenden Ansprüche, wobei die Reaktionserzeugung Sprechen und/oder Schreiben und/oder Bezeichnen einer bevorzugten Option unter den verfügbaren Optionen beinhaltet.

13. Trainingssystem (100) nach einem der vorhergehenden Ansprüche, wobei die Inhaltsverarbeitung Spracherzeugung und/oder Sprachverständnis umfasst oder ist und wobei die Präsentationsfunktionalität (202) dazu konfiguriert ist, die elektronische Steuerungseinheit (1) zu veranlassen, den ersten Präsentationsbefehl auszugeben, um eine Liste kritischer Wörter oder Phrasen über die Benutzerschnittstelle (2, 3) zu präsentieren, um Wörter oder Phrasen anzugeben, bei denen der erste Benutzer (2a) Probleme hat, sie zu erzeugen und/oder zu verstehen.

14. Trainingssystem (100) nach einem der vorhergehenden Ansprüche, wobei die Inhaltsverarbeitung eines oder mehrere oder alle der Folgenden beinhaltet: Inhaltsverständnis, Inhaltsbestätigung, Reaktionserzeugung in Bezug auf den Inhalt.

15. Trainingsanordnung (1000), umfassend:
- das Trainingssystem (100) nach einem der vorhergehenden Ansprüche,
- eine erste Benutzerschnittstelle (2), die dazu konfiguriert ist, eine Eingabe eines ersten Benutzers (2a) zu empfangen und/oder dem ersten Benutzer (2a) ein Feature zu präsentieren,
- eine zweite Benutzerschnittstelle (3), die dazu konfiguriert ist, eine Eingabe eines zweiten Benutzers (3a) zu empfangen und/oder dem zweiten Benutzer (3a) ein Feature zu präsentieren.

16. Trainingsanordnung (1000) nach Anspruch 15, wobei
- die erste (2) und die zweite (3) Benutzerschnittstelle über eine drahtlose Verbindung oder eine Drahtverbindung operativ gekoppelt sind.

## Revendications

1. Système d'entraînement (100) destiné à l'entraînement d'un premier utilisateur (2a) comprenant
a) un produit-programme informatique comprenant un code de programme (P1) qui, lorsqu'il est chargé et exécuté sur une unité de commande électronique (1), fournit un système de commande de fonctionnement (200), et/ou
b) une unité de commande électronique (1) avec un code de programme (P1), ledit code de programme (P1), lorsqu'il est exécuté sur l'unité de commande électronique (1), fournissant un système de commande de fonctionnement (200),
- ledit système de commande de fonctionnement (200) étant configuré pour commander l'entraînement du premier utilisateur (2a),
- ledit système de commande de fonctionnement (200) comprenant une pluralité de fonctionnalités d'entraînement, les fonctionnalités d'entraînement comprenant :
- une fonctionnalité de génération de données (201) configurée pour produire des données d'aptitude d'utilisateur, les données d'aptitude d'utilisateur indiquant l'aptitude du premier utilisateur (2a) dans le traitement de contenu,
ledit contenu comprenant : un contenu lié à la langue, un contenu lié à la mémoire, un contenu lié à la direction, un contenu lié à l'espace visuel, un contenu émotionnel,
ledit contenu comprenant des contenus critiques que le premier utilisateur (2a) a des problèmes à traiter,
- une fonctionnalité de présentation (202) qui est configurée pour amener l'unité de commande électronique (1) à émettre une première commande de présentation en fonction des données d'aptitude d'utilisateur afin de présenter une caractéristique par l'intermédiaire d'une interface utilisateur (2, 3), la caractéristique étant liée à l'aptitude du premier utilisateur (2a) dans le traitement de contenu,
ladite fonctionnalité de présentation (202) étant configurée pour amener l'unité de commande électronique (1) à émettre la première commande de présentation afin de présenter une liste des contenus critiques par l'intermédiaire de l'interface utilisateur (2, 3) pour indiquer des contenus que le premier utilisateur (2a) a des problèmes à traiter.

2. Système d'entraînement (100) selon la revendication 1,
- lesdites fonctionnalités d'entraînement comprenant une fonctionnalité de reconnaissance (203) qui est configurée pour reconnaître un contenu critique en fonction des données d'entrée utilisateur fournies à l'aide d'une interface utilisateur (2, 3) et indiquant une entrée d'un utilisateur (2a, 3a), ledit contenu critique étant un contenu que le premier utilisateur (2a) a des problèmes à traiter,
- ladite fonctionnalité de présentation (202) étant configurée pour amener l'unité de commande électronique (1) à émettre une commande de présentation en fonction du contenu critique reconnu afin de présenter une caractéristique d'assistance par l'intermédiaire d'une interface utilisateur (2, 3), la caractéristique d'assistance étant configurée pour aider le premier utilisateur (2a) à traiter le contenu critique.

3. Système d'entraînement (100) selon la revendication 2,
- ladite fonctionnalité de reconnaissance (203) étant configurée pour reconnaître le contenu critique en fonction des données d'entrée utilisateur fournies à l'aide d'une interface utilisateur (3) et indiquant une entrée d'un second utilisateur (3a).

4. Système d'entraînement (100) selon la revendication 2 ou 3,
- lesdites données d'aptitude d'utilisateur indiquant un ou plusieurs contenus critiques connus pour poser des problèmes au premier utilisateur (2a) pour les traiter,
- ladite fonctionnalité de reconnaissance (203) étant configurée pour reconnaître le contenu critique en fonction des données d'entrée utilisateur et des données d'aptitude d'utilisateur.

5. Système d'entraînement (100) selon l'une quelconque des revendications précédentes,
- ladite fonctionnalité de présentation (202) étant configurée pour amener l'unité de commande électronique (1) à émettre une seconde commande de présentation afin de présenter une liste de contenus non critiques par l'intermédiaire d'une interface utilisateur (2, 3) pour indiquer des contenus que le premier utilisateur (2a) est capable de traiter, et/ou
- ladite première commande de présentation et/ou ladite seconde commande de présentation étant configurées pour présenter la liste de contenus critiques par l'intermédiaire d'une interface utilisateur (3) pour un second utilisateur (3a).

6. Système d'entraînement (100) selon l'une quelconque des revendications précédentes,
- ladite fonctionnalité de présentation (202) étant configurée pour amener l'unité de commande électronique (1) à émettre une commande de présentation afin de présenter une tâche cognitive par l'intermédiaire d'une interface utilisateur (2, 3), la tâche cognitive étant une tâche à réaliser par le premier utilisateur (2a).

7. Système d'entraînement (100) selon l'une quelconque des revendications précédentes,
- lesdites fonctionnalités d'entraînement comprenant une fonctionnalité d'évaluation de performance (208), ladite fonctionnalité d'évaluation de performance (208) étant configurée pour déterminer des données de performance de tâche qui indiquant la performance du premier utilisateur (2a) dans la résolution d'une tâche,
- lesdites fonctionnalités d'entraînement comprenant une fonctionnalité de charge cognitive (205) configurée pour déterminer des données de charge cognitive en fonction des données de performance de tâche et/ou en fonction de données psychophysiologiques,
- lesdites données de charge cognitive indiquant la charge cognitive du premier utilisateur (2a),
- lesdites données psychophysiologiques indiquant au moins une mesure psychophysiologique réalisée sur le premier utilisateur (2a) à l'aide d'un détecteur (5, 5a),
- ledit système de commande de fonctionnement (200) étant configuré pour commander l'entraînement en fonction de la charge cognitive déterminée du premier utilisateur (2a).

8. Système d'entraînement (100) selon la revendication 7,
- ladite fonctionnalité de charge cognitive (205) étant configurée pour déterminer des données de charge cognitive en fonction de l'auto-évaluation fournie par le premier utilisateur (2a).

9. Système d'entraînement (100) selon l'une quelconque des revendications précédentes,
- lesdites fonctionnalités d'entraînement comprenant une fonctionnalité de stimulation (206) qui est configurée pour amener l'unité de commande électronique (1) à émettre une commande de stimulation pour un dispositif de stimulation cérébrale électrique (4, 5) pour amener le dispositif de stimulation cérébrale électrique (4, 5) à réaliser une procédure de stimulation cérébrale électrique,
- ladite fonctionnalité de présentation (202) étant configurée pour amener l'unité de commande électronique (1) à émettre une commande de présentation afin de présenter une tâche d'entraînement à réaliser par le premier utilisateur (2a) par l'intermédiaire d'une interface utilisateur (2, 3),
- ladite fonctionnalité de stimulation (206) comprenant une fonctionnalité de stimulation de tâche pour une stimulation de tâche,
- ladite stimulation de tâche étant configurée pour être synchronisée avec la présentation de la tâche d'entraînement.

10. Système d'entraînement (100) selon l'une quelconque des revendications précédentes,
- lesdites fonctionnalités d'entraînement comprenant une fonctionnalité d'environnement (207) qui est configurée pour recevoir des données d'entrée utilisateur d'environnement indiquant un environnement virtuel sélectionné par l'utilisateur au niveau duquel le premier utilisateur (2a) réalise l'entraînement,
- ladite fonctionnalité de présentation (202) étant configurée pour amener l'unité de commande électronique (1) à émettre une commande de présentation afin de présenter une caractéristique d'environnement sur une interface utilisateur (2, 3), la caractéristique d'environnement indiquant l'environnement virtuel sélectionné par l'utilisateur.

11. Système d'entraînement (100) selon l'une quelconque des revendications précédentes,
- ladite fonctionnalité de génération de données (201) étant configurée pour produire les données d'aptitude d'utilisateur en fonction d'une entrée fournie à l'aide d'une interface utilisateur (2, 3) et réalisées par un utilisateur (2a, 3a).

12. Système d'entraînement (100) selon l'une quelconque des revendications précédentes, ladite production de réponse comprenant la parole et/ou l'écriture et/ou l'indication d'une option préférée parmi les options disponibles.

13. Système d'entraînement (100) selon l'une quelconque des revendications précédentes, ledit traitement de contenu comprenant ou étant une production de langage et/ou une compréhension de langage, et
ladite fonctionnalité de présentation (202) étant configurée pour amener l'unité de commande électronique (1) à émettre la première commande de présentation afin de présenter une liste de mots ou de phrases critiques par l'intermédiaire de l'interface utilisateur (2, 3) pour indiquer des mots ou des phrases que le premier utilisateur (2a) a des problèmes à produire et/ou à comprendre.

14. Système d'entraînement (100) selon l'une quelconque des revendications précédentes, ledit traitement de contenu comprenant une ou plusieurs ou la totalité parmi : la compréhension de contenu, l'accusé de réception de contenu, la production de réponse liée au contenu.

15. Agencement d'entraînement (1000), comprenant :
- le système d'entraînement (100) selon l'une quelconque des revendications précédentes,
- une première interface utilisateur (2) configurée pour recevoir une entrée d'un premier utilisateur (2a) et/ou pour présenter une caractéristique au premier utilisateur (2a),
- une seconde interface utilisateur (3) configurée pour recevoir une entrée d'un second utilisateur (3a) et/ou pour présenter une caractéristique au second utilisateur (3a).

16. Agencement d'entraînement (1000) selon la revendication 15,
- ladite première (2) et ladite seconde (3) interface utilisateur étant fonctionnellement couplées par l'intermédiaire d'une connexion sans fil ou d'une connexion filaire.
